# EUROPEAN PATENT APPLICATION

(11) **EP 4 446 408 A1**
(43) Date of publication of application: **16.10.2024**
(21) Application number: 22904199.1
(22) Date of filing: 05.12.2022
(51) Int. Cl.: C12N 5/071, C07K 14/78, C12M 3/00, C12N 5/0793

(54) **HYDROGEL, INKJET INK, METHOD FOR PRODUCING CELL CULTURE BODY, AND CELL-CONTAINING GEL PARTICLES AND METHOD FOR PRODUCING SAME**

(30) Priority: 06.12.2021 JP 2021197639; 06.12.2021 JP 2021197665
(71) Applicant: Ricoh Company, Ltd., Tokyo 143-8555 (JP)
(72) Inventor: OGIHARA, Miyaka, Tokyo 143-8555 (JP); HEMMI, Natsuko, Tokyo 143-8555 (JP); SATOH, Naoki, Tokyo 143-8555 (JP); YAGINUMA, Hidekazu, Tokyo 143-8555 (JP); YUMOTO, Masayuki, Tokyo 143-8555 (JP); HOSOYA, Toshihiko, Tokyo 143-8555 (JP)
(74) Representative: Marks & Clerk LLP
(86) International application number: PCT/JP2022/044794
(87) International publication number: WO 2023/106271

(57) **Abstract**

An object of the present invention is to provide a hydrogel that has both gel shapeability and cell adhesiveness, and enables a long-time cell culture. Provided is a hydrogel (1) including: a tetra-armed polymer (2) containing polyethylene glycol as a backbone, and containing one or more electrophilic functional groups or nucleophilic functional groups in at least one of a side chain and a terminal; and a linear polymer (3) containing two or more nucleophilic functional groups or electrophilic functional groups in at least one of a side chain and a terminal; the tetra-armed polymer (2) and the linear polymer (3) being cross-linked with each other; wherein the hydrogel is characterized in that the linear polymer (3) is protein, peptide, or polysaccharide, that the electrophilic functional group(s) is/are one or more selected from the group consisting of maleimidyl, N-hydroxy-succinimidyl (NHS), sulfosuccinimidyl, phthalimidyl, imidazoyl, acryloyl, and nitrophenyl, and that the nucleophilic functional group(s) is/are one or more selected from the group consisting of thiol, amino, and -CO₂PhNO₂.

## Description

### Technical Field

The present invention belongs to the technical fields of: a hydrogel; an ink jet ink; a method for producing a cell culture body using the inkjet ink; a cell-containing gel particle and a method for producing the particle; a method for culturing a cell; and a cell-evaluating plate.

### Background Art

There is a demand for a hydrogel composition that can contain a cell in a three-dimensional pattern, and enables a cell to be cultured for a long time. A conventional three-dimensional model formed of a collagen material has cell adhesiveness, but slow in gelation, does not allow prompt gel formation in a culturing place, and thus, causes the position of a cell to be random, making it difficult to achieve the reproducibility of the evaluation result. As an example of a material having good shapeability and prompt in gelation, an alginic acid gel or the like can be taken. In general, however, a gel material having excellent shapeability often does not present cell adhesiveness, makes it difficult to culture a model for a long time, and thus, is limited in use. In some cases, an alginic acid gel or a polymer material is supplemented with a guest material in order to have supplementary cell adhesiveness. For example, (Patent Document 1) discloses a hydrogel composition including: a hydrogel in which tetra-armed polymers having polyethylene glycol as a backbone are mutually cross-linked; and a guest substance; wherein the hydrogel composition is characterized in that the guest substance has a cell adhesion factor such as a collagen peptide. However, adding such a guest material and besides ensuring the amount added and dispersibility often involves high technology, can be a traded-off against shapeability in some cases, and thus, still leaves room for improvement.

By the way, an in vitro cell culture technology involves seeding cells at an optimal seeding density, and culturing the cells under conditions (adhesion/suspension conditions) suited for the cells. When a cell that is difficult to obtain, such as a patient-derived cell, is cultured and assayed, a smaller number of cells are cultured. In general, a method for culturing cells in small numbers is, for example, a single cell culture, spheroid culture, or liquid-phase droplet culture technology. Among these, a cell-encapsulating technology that cultures cells in a hydrogel is already known as a technique that affords a high survival rate, and enables even a smaller number of cells to be easily handled.

However, a culture by a conventional cell-encapsulating technology is often a technique in which, as a hydrogel for encapsulating a cell, alginic acid is used, considering the shapeability. In a hydrogel of alginic acid, however, some cell species have difficulty in surviving even for a short time. For example, (Patent Document 2) discloses a method in which, for the purpose of producing collagen beads having anisotropy, a cell is encapsulated in a hydrogel made by mixing alginic acid and collagen, and quick gelation of the alginic acid allows the shapeability of the gel particles to be maintained until the gelation of the collagen. However, this technology involves bringing the cells into contact with a calcium solution during the gelation, and thus, has difficulty in maintaining the survival rate of the cells.

Another known technique involves using an extracellular matrix such as collagen or gelatin, considering the survival rate of cells. For example, (Patent Document 3) discloses a culture method in which, for the purposes of performing an accurate and sufficient susceptibility test even with a small number of animal cells, and clearly observing and measuring the intercellular interaction during coculture, animal cells are dispersed in a collagen solution, the resulting cell-containing collagen droplets are allowed to gelate on the surface of a support to produce a collagen gel in droplet form, and a culture liquid is added to the gel, in which the animal cells are cultured. With such a method performed utilizing collagen or the like, the survival rate can be maintained, but the shapeability is poor. Accordingly, to maintain the shapeability, the particles containing a cell and a hydrogel are further coated with a hydrogel or another material. Because of this, the particles result in having a large diameter, are difficult to observe, and thus have a problem in that the large particle diameter affects the permeability of the nutrients of the culture liquid, oxygen, and the like in a long-time culture, resulting in decreasing the survival rate. In (Patent Document 2) above, the volume of a collagen gel particle is as large as 3 µl, and the problem of rendering the particle small has not been solved successfully.

Furthermore, if the number of cells encapsulated in a hydrogel is smaller, the survival rate of the cells is more difficult to maintain. That is, there is a problem in that, when a hydrogel material capable of enhancing the shapeability in encapsulation is used in a case where the number of cells per particle is small, the survival rate of cells is not maintained, and on the other hand, that using a hydrogel material capable of enhancing the survival rate of cells causes the shapeability to be poor, causes the particle diameter to be larger, and makes the particle difficult to observe, resulting in failing in a long-time culture.

### Related Art Documents

### [Patent Documents]

[Patent Document 1] Japanese Unexamined Patent Application Publication No. 2020-150846
[Patent Document 2] Japanese Patent No. 6817619
[Patent Document 3] WO 95/18216

### Summary of the Invention

### Technical Problem

Now, an object of the present invention is to provide a hydrogel that has both gel shapeability and cell adhesiveness, and enables a long-time cell culture.

Another object of the present invention is to make it possible to culture a cell, still maintaining the shapeability of a hydrogel containing the cell and the survival rate of the cell, without limitation to the cell species.

### Solution to Problem

The present inventors have made studies vigorously, and consequently completed the invention through the discovery that the above-described problems can be solved by forming a hydrogel through cross-linking between the following: a multibranched polymer that is fast in gelation, and has a specific structure containing polyethylene glycol as a backbone; and a linear polymer such as a protein having cell adhesiveness.

That is, the present invention is a hydrogel including: a multibranched polymer containing polyethylene glycol as a backbone, and containing one or more electrophilic functional groups or nucleophilic functional groups in at least one of a side chain and a terminal; and a linear polymer containing two or more nucleophilic functional groups or electrophilic functional groups in at least one of a side chain and a terminal; the multibranched polymer and the linear polymer being cross-linked with each other; wherein the hydrogel is characterized in that the linear polymer is protein, peptide, or polysaccharide, that the electrophilic functional group(s) is/are one or more selected from the group consisting of maleimidyl, N-hydroxy-succinimidyl (NHS), sulfosuccinimidyl, phthalimidyl, imidazoyl, acryloyl, and nitrophenyl, and that the nucleophilic functional group(s) is/are one or more selected from the group consisting of thiol, amino, and -CO₂PhNO₂.

In addition, the present inventors have completed the invention through the discovery that the above-described problems can be solved by forming a hydrogel for encapsulating a cell, through cross-linking between the following: a multibranched polymer having a specific structure containing polyethylene glycol as a backbone; and a linear polymer, such as a protein, having a specific functional group in at least one of a side chain and a terminal.

That is, the present invention is a cell-containing gel particle including a hydrogel and a cell encapsulated in the hydrogel, the hydrogel including: a multibranched polymer containing polyethylene glycol as a backbone, and containing one or more electrophilic functional groups or nucleophilic functional groups in at least one of a side chain and a terminal; and a linear polymer containing two or more nucleophilic functional groups or electrophilic functional groups in at least one of a side chain and a terminal; the multibranched polymer and the linear polymer being cross-linked with each other; wherein the hydrogel is characterized in that the linear polymer is protein, peptide, or polysaccharide, that the electrophilic functional group(s) is/are one or more selected from the group consisting of maleimidyl, *N*-hydroxy-succinimidyl (NHS), sulfosuccinimidyl, phthalimidyl, imidazoyl, acryloyl, and nitrophenyl, and that the nucleophilic functional group(s) is/are one or more selected from the group consisting of thiol, amino, and -CO₂PhNO₂.

The contents of the disclosures in Japanese Patent Application Nos. 2021-197665 and 2021-197639 which form the basis for priority of the present application are incorporated herein.

### Effects of the Invention

The present invention can provide a hydrogel that has both gel shapeability and cell adhesiveness, and enables a long-time cell culture.

In addition, the present invention makes it possible to culture even one cell, still maintaining the survival rate of the cell, without limitation to the cell species.

### Brief Description of the Drawings

FIG. 1 is a diagram schematically illustrating a hydrogel according to the present embodiment;
FIG. 2 is a schematic diagram illustrating one embodiment of a cell-containing gel particle according to the present invention;
FIG. 3 is a schematic diagram illustrating one embodiment of a method for culturing a cell according to the present invention;
FIG. 4 is a schematic diagram illustrating another embodiment of a method for culturing a cell according to the present invention;
FIG. 5 is a schematic diagram illustrating one embodiment of a cell-evaluating plate according to the present invention;
FIG. 6 is an image of a cell culture body according to Example 1, as observed under a microscope;
FIG. 7 is an image cell culture body according to Example 2, as observed under a confocal microscope;
FIG. 8 is an image of a cell culture body according to Example 3, as observed under a fluorescence microscope;
FIG. 9 is (a) a plan view and (b) a side view of a laminate containing a cell culture body, wherein the laminate was produced in Example 5;
FIG. 10 (a) is a side view of a laminate containing a cell culture body, wherein the laminate was produced in Example 6; FIG. 10 (b) is an enlarged view of the portion A in FIG. 10 (a);
FIG. 11 is an image of a cell culture body according to Example 6, as observed under a microscope;
FIG. 12 is an image of a cell culture body according to Comparative Example 1, as observed under a microscope;
FIG. 13 is an image of a cell culture body according to Comparative Example 2, as observed under a confocal microscope;
FIG. 14 is an image illustrating cell-containing gel particles in Example 7 as a result observed under a phase-contrast microscope;
FIG. 15 is an image illustrating cell-containing gel particles in Example 8 as a result observed under a phase-contrast microscope;
FIG. 16 is an image illustrating cell-containing gel particles in Example 10 as a result observed under a fluorescence microscope;
FIG. 17 is an image illustrating cell-containing gel particles in Example 11 as a result observed under a phase-contrast microscope;
FIG. 18 is an image illustrating cell-containing gel particles in Example 12 as a result observed under a phase-contrast microscope;
FIG. 19 is an image illustrating cell-containing gel particles in Example 13 as a result observed under a phase-contrast microscope; and
FIG. 20 is a graph illustrating the measurement result of the amount of IL-8 with respect to the concentration of LPS in Example 14.

### Mode for Carrying out the Invention

The present invention will be below described in detail with reference to embodiments.

The below-described embodiments are suitable embodiments of the present invention, and thus, are subject to various technically preferable limitations. However, the scope of the present invention is not limited to these aspects, unless otherwise specified to limit the present invention in the description below.

A hydrogel according to the present embodiment is characterized by including: a multibranched polymer containing polyethylene glycol as a backbone, and containing one or more electrophilic functional groups or nucleophilic functional groups in at least one of a side chain and a terminal; and a linear polymer containing two or more nucleophilic functional groups or electrophilic functional groups in at least one of a side chain and a terminal; wherein the multibranched polymer and the linear polymer are cross-linked with each other.

A "hydrogel" refers to a gel having a three-dimensional network structure and configured to retain a large amount of water inside the spaces of the network structure. Preferable examples of the hydrogel include: polysaccharide gels such as agar; protein gels such as jelly; and high water-absorbing polymer gels such as an acrylic acid polymer. In general, a hydrogel is allowed to take in any of various substances in the gel via the water contained therein.

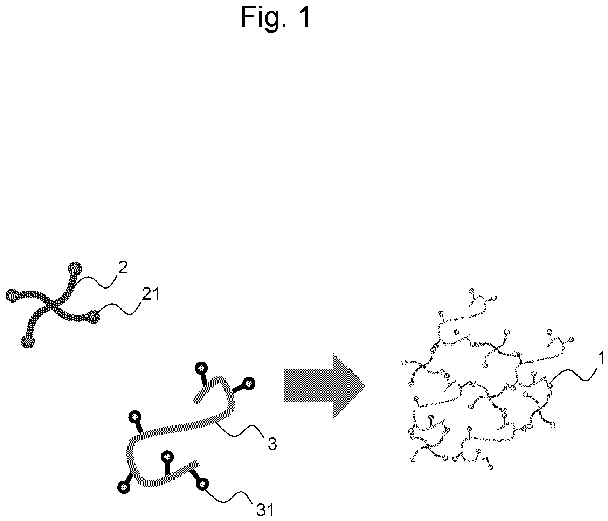

A typical structure of a hydrogel in the present embodiment is schematically illustrated in FIG. 1. As illustrated in FIG. 1, a hydrogel 1 in the present embodiment is formed through cross-linking between the following: an electrophilic functional group 21, for example, a maleimidyl group, of a tetra-armed polymer 2; and a nucleophilic functional group 31, for example, a thiol group, of a linear polymer 3. The hydrogel 1 has a structure in which the tetra-armed polymer and the linear polymer are dispersed. Without limitation to the example in FIG. 1, the tetra-armed polymer may have a nucleophilic functional group, and the linear polymer on the other side may have an electrophilic functional group. The components constituting the hydrogel 1 will be described below.

### <Multibranched Polymer>

A multibranched polymer in the present embodiment is a polymer having at least three polyethylene glycol branches, which are cross-linked with each other to form a network structure. In a preferable aspect, the multibranched polymer is a polymer having four polyethylene glycol branches, and such a tetra-armed polymer containing polyethylene glycol as a backbone is known as a Tetra-PEG. A Tetra-PEG has one or more electrophilic functional groups or nucleophilic functional groups in at least one of a side chain and a terminal. The Tetra-PEG is cross-linked with a linear polymer via these functional groups to construct a network structure. This polymer contains PEG as a main component, and thus, has excellent biocompatibility.

The electrophilic functional group(s) in the multibranched polymer is/are one or more selected from the group consisting of maleimidyl, *N*-hydroxy-succinimidyl (NHS), sulfosuccinimidyl, phthalimidyl, imidazoyl, acryloyl, and nitrophenyl. The electrophilic functional group is preferably a maleimidyl group. In the multibranched polymer, the composition may be the same or different among the electrophilic functional groups, and is preferably the same. The functional groups having the same composition have uniform reactivity with the nucleophilic functional groups of a linear polymer to form cross-linking bonds, and thus making it easier to obtain a gel having a uniform stereostructure.

Examples of the multibranched polymer having one or more maleimidyl groups in at least one of a side chain and a terminal, and containing polyethylene glycol as a backbone include a compound having a maleimidyl group in a terminal, and represented by the following Formula (I).

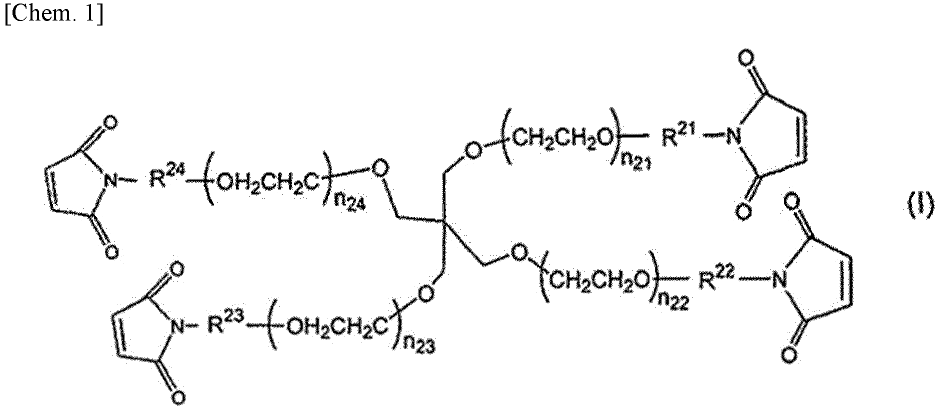

In Formula (I), n₂₁ to n₂₄ may be the same or different. n₂₁ to n₂₄ having values closer to each other enable the gel to have a more uniform stereostructure, and have higher strength, and thus, are more preferable. The values are particularly preferably the same. n₂₁ to n₂₄ having an excessively high value cause the strength of the gel to be weak. n₂₁ to n₂₄ having an excessively low value cause the formation of a gel to be made difficult by the steric hindrance of the compound. Accordingly, n₂₁ to n₂₄ suitably have a value of 5 or more and 300 or less, preferably 20 or more and 250 or less, more preferably 30 or more and 180 or less, still more preferably 45 or more and 115 or less, particularly preferably 45 or more and 55 or less. The weight-average molecular weight of the multibranched polymer having an electrophilic functional group is preferably 5 × 10³ or more and 5 × 10⁴ or less, more preferably 7.5 × 10³ or more and 3 × 10⁴ or less, still more preferably 1 × 10⁴ or more and 2 × 10⁴ or less.

In Formula (I), R²¹ to R²⁴ are linker sites that link a functional group and a core moiety. R₂₁ to R₂₄ may be the same or different, and are preferably the same to produce a high-strength gel having a uniform stereostructure. In Formula (I), R²¹ to R²⁴ are the same or different, and are C₁-C₇ alkylene, C₂-C₇ alkenylene, -NH-R²⁵-, -CO-R²⁵-, -R²⁶-O-R²⁷-, -R²⁶-NH-R²⁷-, -R²⁶-CO₂-R²⁷-, -R²⁶-CO₂-NH-R²⁷-, -R²⁶-CO-R²⁷-, -R²⁶-NH-CO-R²⁷-, -R²⁶-CO-NH-R²⁷-, or the like. Here, R²⁵ represents C₁-C₇ alkylene. R²⁶ represents C₁-C₃ alkylene. R²⁷ represents C₁-C₅ alkylene.

In addition, the nucleophilic functional group(s) in the multibranched polymer is/are one or more selected from the group consisting of thiol, amino, and -CO₂PhNO₂. Here, Ph represents an *o*-, *m-,* or *p*-phenylene group. In the multibranched polymer, the composition may be the same or different among the nucleophilic functional groups, and is preferably the same. The functional groups having the same composition have uniform reactivity with the electrophilic functional groups of a linear polymer to form cross-linking bonds, and thus make it easier to obtain a gel having a uniform stereostructure.

Examples of the multibranched polymer having one or more thiol groups in a side chain and a terminal, and containing polyethylene glycol as a backbone include a compound having a thiol group in a terminal, and represented by the following Formula (II).

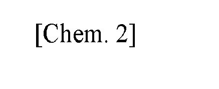

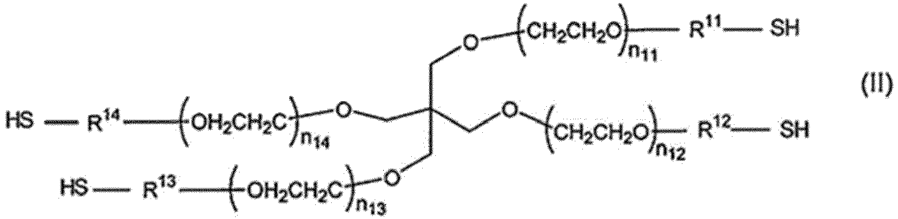

In Formula (II), n₁₁ to n₁₄ may be the same or different. n₁₁ to n₁₄ having values closer to each other enable a more uniform stereostructure, have higher strength, and thus, are more preferable. The values are particularly preferably the same. n₁₁ to n₁₄ having an excessively high value cause the strength of the gel to be weak. n₁₁ to n₁₄ having an excessively low value cause the formation of a gel to be made difficult by the steric hindrance of the compound. Accordingly, n₁₁ to n₁₄ preferably have a value of 25 or more and 250 or less, more preferably 35 or more and 180 or less, still more preferably 50 or more and 115 or less, particularly preferably 50 or more and 60 or less. The weight-average molecular weight of the multibranched polymer having a nucleophilic functional group is preferably 5 × 10³ or more and 5 × 10⁴ or less, more preferably 7.5 × 10³ or more and 3 × 10⁴ or less, still more preferably 1 × 10⁴ or more and 2 × 10⁴ or less.

In Formula (II), R¹¹ to R¹⁴ are linker sites that link a functional group and a core moiety. R¹¹ to R¹⁴ may be the same or different, and is preferably the same to produce a high-strength gel having a uniform stereostructure. In Formula (II), R¹¹ to R¹⁴ are the same or different, and is C₁-C₇ alkylene, C₂-C₇ alkenylene, -NH-R¹⁵-, -CO-R¹⁵-, -R¹⁶-O-R¹⁷-, -R¹⁶-NH-R¹⁷-, -R¹⁶-CO₂-R¹⁷-, -R¹⁶-CO₂-NH-R¹⁷-, -R¹⁶-CO-R¹⁷-, -R¹⁶-NH-CO-R¹⁷-, -R¹⁶-CO-NH-R¹⁷-, or the like. Here, R¹⁵ represents C₁-C₇ alkylene. R¹⁶ represents C₁-C₃ alkylene. R¹⁷ represents C₁-C₅ alkylene.

Here, "C₁-C₇ alkylene" means an optionally branched alkylene group having one or more and seven or less carbon atoms, and means a linear C₁-C₇ alkylene group or a C₂-C₇ alkylene group having one or more branches (the number of the carbon atoms including the carbon atoms in the branch(es) is one or more and seven or less). Examples include methylene, ethylene, propylene, and butylene. More specific examples include -CH₂-, -(CH₂)₂-, -(CH₂)₃-, - CH(CH₃)-, -(CH₂)₃-, -(CH(CH₃))₂-, -(CH₂)₂-CH(CH₃)-, -(CH₂)₃-CH(CH₃)-, -(CH₂)₂-CH(C₂H₅)-, -(CH₂)₆-, -(CH₂)₂-C(C₂H₅)₂-, and -(CH₂)₃C(CH₃)₂CH₂-.

"C₂-C₇ alkenylene" is a linear or branched alkenylene group having two to seven carbon atoms, and having one or more double bonds in a chain. The alkenylene is, for example, a divalent group having a double bond, obtained by removing two to five of the hydrogen atoms at the coadjacent carbon atoms from the alkylene group.

Herein, an alkylene group and an alkenylene group may have any one or more substituents. Examples of such a substituent include, but are not limited to, alkoxy, halogen atom (which may be any one of a fluorine atom, chlorine atom, bromine atom, and iodine atom), amino, mono- or di-substituted amino, substituted silyl, acyl, or aryl. When two or more substituents are present, the substituents may be the same or different.

In addition, when a functional group is herein defined as a group that "may have a substituent", the kind of the substituent, the position of substitution, and the number of substituents are not particularly limited. When two or more substituents are present, the substituents may be the same or different. Examples of the substituent include, but are not limited to, alkyl, alkoxy, hydroxyl, carboxyl, halogen atom, sulfo, amino, alkoxy carbonyl, and oxo. These substituents may further contain a substituent.

### <Linear Polymer>

The linear polymer that forms a hydrogel through a cross-linking reaction with a multibranched polymer contains two or more nucleophilic functional groups or electrophilic functional groups in at least one of a side chain and a terminal. For cross-linking, the linear polymer has a nucleophilic functional group when the multibranched polymer has an electrophilic functional group, and the linear polymer has an electrophilic functional group when the multibranched polymer has a nucleophilic functional group. The details about the electrophilic functional group and the nucleophilic functional group are the same as the details about the above-described multibranched polymer.

The molar ratio of an electrophilic functional group to a nucleophilic functional group in the multibranched polymer and the linear polymer is preferably in the range of from 0.5:1 to 1.5:1. The respective functional group components react at a molar ratio of 1:1 to be cross-linked, and thus, the mixing molar ratio is preferably closer to 1:1. To obtain a high-strength hydrogel, the ratio of the nucleophilic functional group: the electrophilic functional group is particularly preferably in the range of from 0.8:1 to 1.2:1. These functional groups are still more preferably present in the respective terminals of the multibranched polymer and the linear polymer. In a preferable aspect, it is also possible that two or more different kinds of gel precursors having different mixing ratios are once formed, and then, such gel precursors are further cross-linked to obtain a hydrogel of interest.

The linear polymer is a protein, peptide, or polysaccharide. The protein is not particularly limited, and can be suitably selected according to the purpose. Examples include collagen, fibrin, albumin, fibronectin, laminin, tenascin, entactin, elastin, and compounds derived therefrom. In particular, gelatin is preferably used as the linear polymer. In addition, a peptide is two or more amino acid residues polymerized via a peptide bond (amide bond). Examples of a peptide that is usable include a collagen peptide. The peptide may be any one of a dipeptide, tripeptide, oligopeptide (containing approximately 10 amino acids), and polypeptide (containing tens to hundreds of amino acids). Furthermore, the polysaccharide is not particularly limited, and can be suitably selected. Examples include cellulose, chitin, chitosan, hyaluronic acid, alginic acid, starch, and pectin. These linear polymers may be used singly or in combination of two or more kinds thereof. In addition, a complex of a protein, such as proteoglycan, and sugar can be used. The molecular weight of the linear polymer is not particular limited as long as the molecular weight is in the range in which the linear polymer can be present inside a hydrogel. The molecular weight can be preferably 100 to 500000. A linear polymer containing two or more nucleophilic functional groups or electrophilic functional groups in at least one of a side chain and a terminal is illustrated below with reference to a gelatin having a thiol group in a side chain.

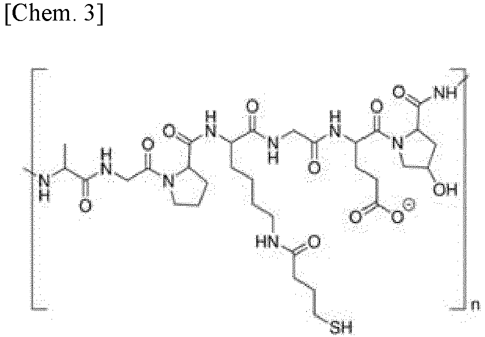

### <Cell>

In the present embodiment, a hydrogel can contain a cell. The hydrogel in the present embodiment has excellent gel shapeability, and also has cell adhesiveness due to the linear polymer. In the hydrogel, a cell can be cultured for a long time. The species or the like of the cell is not particularly limited, and can be suitably selected according to the purpose. Any species of cell can be used, taxonomically irrespective of whether the cell is, for example, a eukaryotic cell, prokaryotic cell, multicellular organism cell, or unicellular organism cell.

Examples of the eukaryotic cell include animal cells, insect cells, plant cells, and fungi. These may be used singly or in combination of two or more species thereof. Among these, animal cells are preferable. In a case where the cells form a cell aggregate, adhesive cells adherent to each other, and having such cell adhesiveness as to be not isolated, unless physicochemically treated, are more preferable.

The adhesive cell is not particularly limited, and can be suitably selected according to the purpose. Examples include differentiated cells and undifferentiated cells.

Examples of the differentiated cell include: a hepatocyte that is a parenchymal cell of liver; stellate cell; Kupffer cell; vascular endothelial cell; endothelial cell such as sinusoidal endothelial cell or corneal endothelial cell; fibroblast; osteoblast; osteoclast; periodontiumderived cell; epidermal cell such as epidermal keratinocyte; tracheal epithelial cell; gastrointestinal epithelial cell; uterocervical epithelial cell; epithelial cell such as corneal epithelial cell; lactocyte; pericyte; myocyte such as smooth muscle myocyte or cardiomyocyte; nephrocyte; pancreas islet cell; neurocyte such as peripheral neurocyte or optic nerve cell; chondrocyte; and osteocyte. For a hydrogel containing a neurocyte, the adhesive cell may be a primary cell collected directly from a tissue or an organ, or may be a cell obtained by subj ecting the primary cell to a passage(s).

The undifferentiated cell is not particularly limited, and can be suitably selected according to the purpose. Examples include: an embryonic stem cell that is an undifferentiated cell; a pluripotent stem cell such as a mesenchymal stem cell having pluripotency; a unipotent stem cell such as an endothelial precursor cell having unipotency; and an iPS cell.

### Examples of the prokaryotic cell include eubacteria and archaea.

Specific examples of the cell include a normal human dermal fibroblast. As the normal human dermal fibroblast, a commercially available product can be used. Examples of the commercially available product include a product under the tradename CC2507 (manufactured by Lonza Group AG).

In addition, the cell to be contained may be a cell aggregate (spheroid) that has preliminarily undergone a culture step. In a hydrogel containing a cell aggregate, maturation and differentiation can occur better than in a hydrogel in which a cell is dispersed. The size of the cell aggregate is not particularly limited. The diameter is preferably, for example, 100 µm or less.

The cell(s) that can be contained is at least one or more species of cells. In a hydrogel containing two or more species of cells, a cell culture body in which the functional expression of the cell is more promoted can be produced by interaction between the cell species than by a single cell species.

The hydrogel that encapsulates a cell may be supplemented with an extracellular matrix, if desired. The extracellular matrix is not particularly limited, and can be suitably selected according to the purpose. Examples include: extracellular matrix proteins and sugar proteins, such as collagen, laminin, fibronectin, elastin, fibrin, proteoglycan, hyaluronic acid, heparan sulfate proteoglycan, and chondroitin sulfate proteoglycan; and proliferators and growth factors, such as hepatocyte proliferators, fibroblast proliferators, and nerve growth factors, depending on the cell. These may be used singly or in combination of two or more kinds thereof. It is known that such an extracellular matrix promotes the adhesion, proliferation, or differentiation of a cell. Contained in a hydrogel, the extracellular matrix can function as a trigger that changes the form a cell in the hydrogel formed.

Such a hydrogel as above-described can be formed from an ink including: a first solution including a multibranched polymer containing polyethylene glycol as a backbone, and containing one or more electrophilic functional groups or nucleophilic functional groups in at least one of a side chain and a terminal; and a second solution including a linear polymer containing two or more nucleophilic functional groups or electrophilic functional groups in at least one of a side chain and a terminal. Specifically, mixing the first solution and the second solution that are thereby cross-linked can afford a hydrogel. In addition, it is possible that the second solution is mixed in the first solution, and then, the first solution and the second solution are mixed. The amount of the second solution to be preliminarily mixed in can be suitably selected to the extent that neither thickening nor gelation occurs noticeably. It is also possible that the first solution is mixed in the second solution, and then, the first solution and the second solution are mixed.

The above-described ink is used as an ink jet ink. One of the first solution and the second solution is discharged by an ink jet method, and landed on the other one of the second solution and the first solution, whereby the first solution and the second solution can be mixed, forming a hydrogel. The second solution or the first solution as a target of landing may be, for example, a liquid filled into a container, or may be a liquid film applied to a base material preliminarily. Alternatively, the solution may be in the form of droplets discharged onto a base material preliminarily by an inkjet method, or in the form of a liquid film that is formed by contact between or combination of many droplets discharged continuously, and has any shape, such as a linear shape. Here, "ink jet" refers to a means by which a solution contained in a liquid chamber is injected in the form of droplets through the discharge hole (nozzle) of an ink jet head to be landed on a target site. The discharge method enables a fine solution (droplet) to be discharged through the discharge hole, and thus, can produce a highly accurate three-dimensional structure. Using an ink jet method enables the first solution and the second solution to be mixed, for example, at a resolution of 500 µm or less.

When this is conducted, any one or both of the first solution and the second solution of an ink jet ink can contain the above-described cell. For example, onto the second solution containing no cell on a base material, the first solution containing a cell is discharged by an ink jet method, whereby a hydrogel (cell culture body) containing a cell can be formed.

When a cell culture body is produced from an ink jet ink containing a cell, the first solution and the second solution can be mixed on a base material having a patterned structure on the surface. Examples of the patterned structure include an uneven structure formed with a cyclical pattern or any other pattern. In this regard, the patterned structure may be formed in such a manner that the surface itself on the base material has a pattern such as is uneven. For example, the above-described hydrogel containing no cell may be arranged with a predetermined pattern on the surface of a flat base material. Forming a cell culture body in the form of a patterned structure makes it possible, for example, to experiment on how the wandering and spreading state of a cell in a hydrogel is affected by the patterned structure.

In addition, a cell culture body formed from an inkjet ink containing a cell is heated, if desired, and brought into contact with another cell culture body, thus enabling the cell culture bodies to be combined with each other. The temperature for the heating depends on the composition of the hydrogel and the species of the cell. There is no particular problem if the temperature does not cause an excessive damage to the cell contained in the cell culture body. The temperature is preferably, for example, 0°C or more and 40°C or less. The temperature may be raised to 40°C or more, if partially and in a very short time.

The present invention also relates to a method for producing a cell culture body, including: retaining a first solution including a multibranched polymer containing polyethylene glycol as a backbone, and having one or more functional groups in at least one of a side chain and a terminal, any of the functional group(s) being one of a nucleophilic functional group and an electrophilic functional group; and landing droplets of a second solution on the first solution to form one or more hydrogels, the droplets being discharged from a droplet-discharging device in such a manner that the second solution comes into contact with the first solution, the second solution including a linear polymer having one or more functional groups in at least one of a side chain and a terminal, any of the functional group(s) being the other one of the nucleophilic functional group and the electrophilic functional group. Here, the multibranched polymer and the linear polymer are as above-described in reference to a hydrogel.

In addition, at least one of the first solution and the second solution may contain a cell. The cell to be contained is as above-described.

The retaining step is a step of retaining the first solution on a support, a hydrogel, or the like. For example, a support may be immersed in the first solution so that the support can be impregnated with the first solution, which is thus retained on the whole surface of and/or inside of the support. Alternatively, the first solution may be discharged from a droplet-discharging device by a discharging method in such a manner that the first solution is firmly attached and retained at a specific position on the surface of a support or the surface of the hydrogel. The discharging method is, for example, an inkjet method described in detail in reference to the next step. In addition, the first solution may be retained using a cell layer as a support. Here, a "support" is a member that retains the first solution. The material of the support is not particularly limited as long as the material is not such as to inhibit the activity and proliferation of a cell. The material can be suitably selected according to the purpose. The material is preferably a material to which a cell can be firmly attached, or a material that can easily adsorb a cell-adhesive material.

The support can be roughly classified into an organic material or an inorganic material. Examples of the organic material include synthetic resins, silicone-based materials, natural resins, cellulose structures (wood, paper, and the like), chitin structures, and natural fibers (silk, fur, cotton, spongin fiber, and the like). In addition, the cell layer firmly attached to a base plate or the like can be an organic-material support. Examples of the synthetic resin include polyethylene terephthalate (PET), polystyrene (PS), polycarbonate (PC), TAC (triacetyl cellulose), polyimide (PI), nylon (Ny), low-density polyethylene (LDPE), medium-density polyethylene (MDPE), vinyl chloride, vinylidene chloride, polyphenylene sulfide, polyethersulfone, polyethylene naphthalate, polypropylene, or an acryl-based material such as urethane acrylate. Example of the silicone-based material include polydimethyl siloxane (PDMS). Examples of the inorganic material include glass (including glass fiber), earthenware (ceramic, enamel, and the like), metal, carbon fiber, and calcium phosphate structures (bone, teeth, seashell, and the like).

The above-described materials may be used singly, or two or more kinds of organic materials, inorganic materials, or organic materials and inorganic materials may be used in combination. Examples include a fiber-reinforced plastic (FRP) in which carbon fiber or glass fiber and synthetic resin are combined.

The size of the support is not particularly limited. The size can be suitably selected according to the purpose. The shape of the support is not particularly limited, and can be suitably selected according to the purpose. The support may be, for example, in the shape of a solid, such as a dish, multiplate, flask, membrane, or cell insert, or may be in the shape of a flat plate or a flat membrane, such as a glass plate, slide glass, or cover glass.

The structure of the support is not particularly limited, and can be suitably selected according to the purpose. Examples include a porous structure, mesh structure, uneven structure, and honeycomb structure. The porous structure, mesh structure, or the like can retain a large amount of a solution, and thus, is particularly preferable as a support.

The gel-forming step is a step in which the second solution is landed from a droplet-discharging device so as to come into contact with the first solution retained in the retaining step, whereby the first solution and the second solution are mixed, so that a nucleophilic functional group and an electrophilic reactive group of the multibranched polymer and the linear polymer contained in the respective solutions react to be bound to each other, forming a hydrogel. The phrase "so as to come into contact" means that the first solution and the second solution are mixed through contact.

Here, a "droplet-discharging device" refers to a means by which a solution contained in a liquid chamber is injected in the form of droplets to be landed on a target site. Examples of the discharge method of the discharging device include an inkjet method, gel extrusion method, and dispenser method. In an ink jet method, a solution is injected through a discharge hole (nozzle). The discharge method enables a fine solution (referred to as a droplet in some cases) to be discharged through the discharge hole, and thus, can produce a highly accurate three-dimensional structure. When this is conducted, a droplet discharged from a droplet-discharging device may contain a cell or no cell.

The amount of the droplets to be discharged can be any amount. The amount is preferably 9 pL or more, 15 pL or more, 20 pL or more, 30 pL or more, 40 pL or more, 50 pL or more, 60 pL or more, 70 pL or more, 80 pL or more, 90 pL or more, or 100 pL or more, and 900 pL or less, 800 pL or less, 700 pL or less, 600 pL or less, 500 pL or less, 400 pL or less, or 300 pL or less.

The "landing" refers to bringing a solution into contact with a target site. This is achieved by discharging a droplet against a target site by a discharge method.

When the second solution contains a cell, the number of the cells is preferably 5 × 10⁵ cells/mL to 1 × 10⁸ cells/mL, more preferably 1 × 10⁶ cells/mL to 5 × 10⁷ cells/mL. The cell density, if lower than these values, makes it difficult to form a hydrogel containing a suitable number of cells, and contrarily, the cell density, if higher, makes it difficult to discharge a solution by a discharge method such as an inkjet method.

The position at which the solution is discharged is not particularly limited. The solution may be discharged in such a manner that the solution is landed at a desired target position. In addition, the landing sites may be apart from each other, or some of the sites may be in contact or overlap with each other.

The gel is formed through reaction between the polymers having a nucleophilic functional group and an electrophilic functional group, and thus, allowing the second solution to land on the first solution forms a hydrogel. When the second solution is discharged from a droplet-discharging device, a hydrogel formed by a single landing can usually be in the form of a dotted hydrogel, without limitation. As used herein, the phrase "dotted (shape)" refers to a point-like shape. Accordingly, the shape is not limited to a completely circular shape or a hemispheric shape, and may be any of an approximate circular shape, generally hemispheric shape, polygonal shape, generally polygonal shape, amorphous shape, and a combination thereof. In addition, the dotted shape may have a predetermined length and thickness in terms of a three-dimensional structure. When the second solution is discharged a plurality of times, hydrogels having various shapes are formed, in which a dotted hydrogel is the smallest unit.

The volume of a hydrogel formed through a cross-linking reaction by a single landing, that is, a dotted hydrogel depends on the number of discharges of the second solution to the same site. In addition, the larger the diameter of the discharge hole, and the larger the number of discharges to the same site, the larger the volume of the dotted hydrogel. Accordingly, changing the number of discharges to the same site and the diameter of the discharge hole enables the volume of the dotted hydrogel to be adjusted. Without limitation, it is preferable that the volume of the dotted hydrogel herein is 9 pL or more, 15 pL or more, 20 pL or more, 30 pL or more, 40 pL or more, 50 pL or more, 60 pL or more, 70 pL or more, 80 pL or more, 90 pL or more, or 100 pL or more, and 900 pL or less, 800 pL or less, 700 pL or less, 600 pL or less, 500 pL or less, 400 pL or less, or 300 pL or less. Without limitation, the diameter of the dotted hydrogel may be in the range of 10 µm or more and 300 µm or less, and the thickness may be in the range of 5 µm or more and 150 µm or less.

In the present step, the second solution is discharged an arbitrary number of times in one step, and thus, a plurality of dotted hydrogels are formed in some cases. All or part of the dotted hydrogels may be in contact with each other. Allowing a plurality of dotted hydrogels to be arranged in a row not only can form dotted hydrogels but also can form hydrogels in any shape. The shape can be suitably selected according to the purpose. For example, arranging the dots in a uniaxial direction allows forming a linear hydrogel. In addition, arranging linear hydrogels in the form of one plane without a gap therebetween allows forming a film-like (sheetlike) hydrogel. Controlling the fusion and separation between the dotted hydrogels in the linear or film-like hydrogel formed allows controlling or inhibiting the migration and spreading of cells contained in each dotted hydrogel. Further, forming a film-like hydrogel enables gels to be laminated easily.

In addition, when the solution contains cells, the number of the cells contained in a dotted hydrogel depends on the concentration of the cells in the solution, and thus, the number of times when a dotted hydrogel is formed can be used to control the cell density.

### <Culture Medium>

A cell culture body produced is placed in a suitable culture medium, whereby a cell in a hydrogel can be cultured for a long time. A culture medium is a solution that contains a component for maintaining the shape of a cell culture body, and maintaining the survival of a cell, prevents drying, and conditions the external environment such as an osmotic pressure. The culture medium is not particularly limited, and can be suitably selected from known culture media according to the usage.

Examples of the culture medium include: culture media classified according to the composition, such as a natural medium, semisynthetic medium, and synthetic medium; culture media classified according to the shape, such as a semisolid medium, liquid medium, and powdery medium (hereinafter referred to as a "powder medium" in some cases). These may be used singly or in combination of two or more kinds thereof. Any cell derived from an animal can be used as long as the cell is a culture medium usable to culture an animal cell.

The culture medium to be used to culture an animal cell is not particularly limited, and can be suitably selected according to the purpose. Examples include Dulbecco's Modified Eagles's medium (D-MEM), Ham's F12 medium (Ham's Nutrient Mixture F12), D-MEM/F12 medium, McCoy's 5A medium, Eagles's MEM medium (Eagles's Minimum Essential Medium; EMEM), αMEM medium (alpha Modified Eagles's Minimum Essential Medium; αMEM), MEM medium (Minimum Essential Medium), RPMI1640 medium, Iscove's Modified Dulbecco's medium (IMDM), MCDB131 medium, William's medium E, IPL41 medium, Fischer's medium, StemPro-34 (manufactured by Invitrogen), X-VIVO 10 (manufactured by Cambrex Corporation), X-VIVO 15 (manufactured by Cambrex Corporation), HPGM (manufactured by Cambrex Corporation), StemSpan H3000 (manufactured by Stem Cell Technology Inc.), StemSpan SFEM (manufactured by Stem Cell Technology Inc.), Stemline II (manufactured by Sigma-Aldrich), QBSF-60 (manufactured by Quality Biological Inc.), StemPro hESC SFM (manufactured by Invitrogen), Essential 8 (registered trademark) medium (manufactured by Gibco), Stemfit AK02N (manufactured by Ajinomoto Co., Inc.), Stemfit Basic 02 (manufactured by Ajinomoto Co., Inc.), mTeSR 1 or 2 medium (manufactured by Stem Cell Technology Inc.), ReproFF or ReproFF2 (manufactured by Reprocell Inc.), PSGro hESC/iPSC medium (manufactured by System Biosciences, LLC), NutriStem (registered trademark) medium (manufactured by Biological Industries Ltd.), CSTI-7 medium (manufactured by Cell Science & Technology Institute, Inc.), MesenPRO RS medium (manufactured by Gibco), MF-Medium (registered trademark) mesenchymal stem cell growth medium (manufactured by Toyobo Co., Ltd.), Sf-900II (manufactured by Invitrogen), and Opti-Pro (manufactured by Invitrogen). These may be used singly or in combination of two or more kinds thereof.

The carbon dioxide concentration of a culture medium is not particularly limited, can be suitably selected according to the purpose, and is preferably 2% or more and 5% or less. Having the carbon dioxide concentration at 2% or more and 5% or less enables a cell to be cultured suitably.

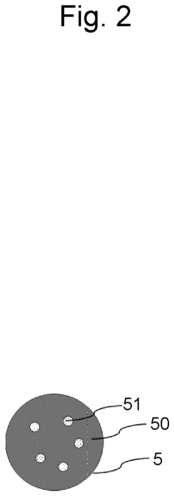

Next, one embodiment of a cell-containing gel particle according to the present invention will be described with reference to FIG. 2. As illustrated in FIG. 2, a cell-containing gel particle 5 according to the present embodiment includes a hydrogel 50 and a cell 51 encapsulated in the hydrogel, the hydrogel including: a multibranched polymer containing polyethylene glycol as a backbone, and containing one or more electrophilic functional groups or nucleophilic functional groups in at least one of a side chain and a terminal; and a linear polymer containing two or more nucleophilic functional groups or electrophilic functional groups in at least one of a side chain and a terminal; the multibranched polymer and the linear polymer being cross-linked with each other. The components constituting the cell-containing gel particle 5 will be described below.

### <Multibranched Polymer>

A multibranched polymer in the present embodiment is a polymer having a plurality of polyethylene glycol branches, which are cross-linked with each other to form a network structure. In a preferable aspect, the multibranched polymer has four polyethylene glycol branches, and such a tetra-armed polymer containing polyethylene glycol as a backbone is known as a Tetra-PEG. A Tetra-PEG has one or more electrophilic functional groups or nucleophilic functional groups in at least one of a side chain and a terminal. The Tetra-PEG is cross-linked with a linear polymer via these functional groups to construct a network structure.

The electrophilic functional group(s) in the multibranched polymer is/are one or more selected from the group consisting of maleimidyl, *N*-hydroxy-succinimidyl (NHS), sulfosuccinimidyl, phthalimidyl, imidazoyl, acryloyl, and nitrophenyl. In the multibranched polymer, the composition may be the same or different among the electrophilic functional groups, and is preferably the same. The functional groups having the same composition have uniform reactivity with the nucleophilic functional groups of a linear polymer to form cross-linking bonds, and thus make it easier to obtain a gel having a uniform stereostructure.

Examples of the multibranched polymer having one or more maleimidyl groups in at least one of a side chain and a terminal, and containing polyethylene glycol as a backbone include a compound having a maleimidyl group in a terminal, and represented by the following Formula (I).

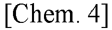

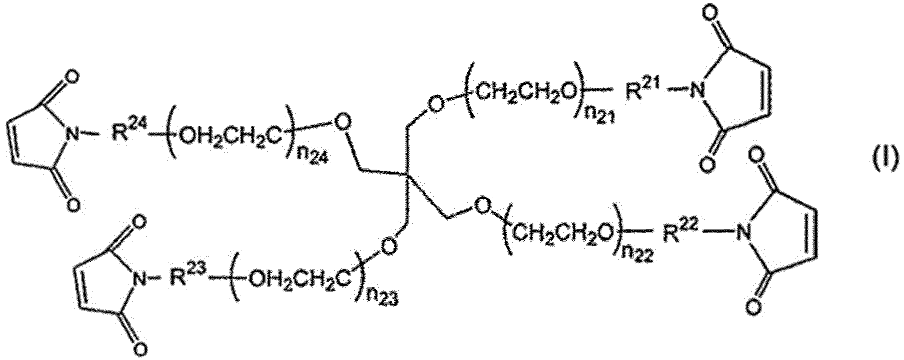

In Formula (I), n₂₁ to n₂₄ may be the same or different. n₂₁ to n₂₄ having values closer to each other enable the gel to have a more uniform stereostructure, and have higher strength, and thus, are more preferable. The values are particularly preferably the same. n₂₁ to n₂₄ having an excessively high value cause the strength of the gel to be weak. n₂₁ to n₂₄ having an excessively low value cause the formation of a gel to be made difficult by the steric hindrance of the compound. Accordingly, n₂₁ to n₂₄ suitably have a value of 5 or more and 300 or less, preferably 20 or more and 250 or less, more preferably 30 or more and 180 or less, still more preferably 45 or more and 115 or less, particularly preferably 45 or more and 55 or less. The weight-average molecular weight of the multibranched polymer having an electrophilic functional group is preferably 5 × 10³ or more and 5 × 10⁴ or less, more preferably 7.5 × 10³ or more and 3 × 10⁴ or less, still more preferably 1 × 10⁴ or more and 2 × 10⁴ or less.

In Formula (I), R²¹ to R²⁴ are linker sites that link a functional group and a core moiety. R₂₁ to R₂₄ may be the same or different, and are preferably the same to produce a high-strength gel having a uniform stereostructure. In Formula (I), R²¹ to R²⁴ are the same or different, and are C₁-C₇ alkylene, C₂-C₇ alkenylene, -NH-R²⁵-, -CO-R²⁵-, -R²⁶-O-R²⁷-, -R²⁶-NH-R²⁷-, -R²⁶-CO₂-R²⁷-, -R²⁶-CO₂-NH-R²⁷-, -R²⁶-CO-R²⁷-, -R²⁶-NH-CO-R²⁷-, -R²⁶-CO-NH-R²⁷-, or the like. Here, R²⁵ represents C₁-C₇ alkylene. R²⁶ represents C₁-C₃ alkylene. R²⁷ represents C₁-C₅ alkylene.

In addition, the nucleophilic functional group(s) in the multibranched polymer is/are one or more selected from the group consisting of thiol, amino, and -CO₂PhNO₂. In the multibranched polymer, the composition may be the same or different among the nucleophilic functional groups, and is preferably the same. The functional groups having the same composition have uniform reactivity with the electrophilic functional groups of a linear polymer to form cross-linking bonds, and thus making it easier to obtain a gel having a uniform stereostructure.

Examples of the multibranched polymer having one or more thiol groups in a side chain and a terminal, and containing polyethylene glycol as a backbone include a compound having a thiol group in a terminal, and represented by the following Formula (II).

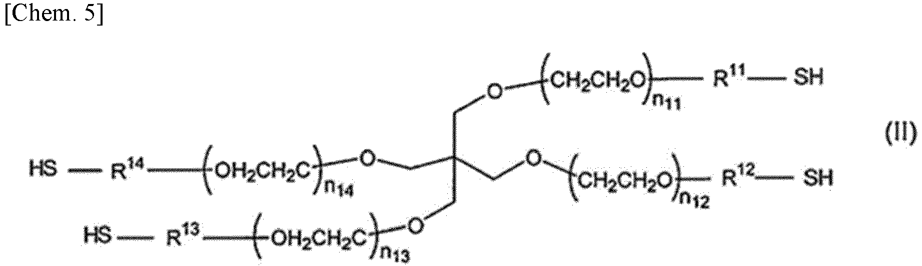

In Formula (II), n₁₁ to n₁₄ may be the same or different. n₁₁ to n₁₄ having values closer to each other enable a more uniform stereostructure, have higher strength, and thus, are more preferable. The values are particularly preferably the same. n₁₁ to n₁₄ having an excessively high value cause the strength of the gel to be weak. n₁₁ to n₁₄ having an excessively low value cause the formation of a gel to be made difficult by the steric hindrance of the compound. Accordingly, n₁₁ to n₁₄ preferably have a value of 25 or more and 250 or less, more preferably 35 or more and 180 or less, still more preferably 50 or more and 115 or less, particularly preferably 50 or more and 60 or less. The weight-average molecular weight of the multibranched polymer having a nucleophilic functional group is preferably 5 × 10³ or more and 5 × 10⁴ or less, more preferably 7.5 × 10³ or more and 3 × 10⁴ or less, still more preferably 1 × 10⁴ or more and 2 × 10⁴ or less.

In Formula (II), R¹¹ to R¹⁴ are linker sites that link a functional group and a core moiety. R¹¹ to R¹⁴ may be the same or different, and are preferably the same to produce a high-strength gel having a uniform stereostructure. In Formula (II), R¹¹ to R¹⁴ are the same or different, and are C₁-C₇ alkylene, C₂-C₇ alkenylene, -NH-R¹⁵-, -CO-R¹⁵-, -R¹⁶-O-R¹⁷-, -R¹⁶-NH-R¹⁷-, -R¹⁶-CO₂-R¹⁷-, -R¹⁶-CO₂-NH-R¹⁷-, -R¹⁶-CO-R¹⁷-, -R¹⁶-NH-CO-R¹⁷-, -R¹⁶-CO-NH-R¹⁷-, or the like. Here, R¹⁵ represents C₁-C₇ alkylene. R¹⁶ represents C₁-C₃ alkylene. R¹⁷ represents C₁-C₅ alkylene.

Here, "C₁-C₇ alkylene" means an optionally branched alkylene group having one or more and seven or less carbon atoms, and means a linear C₁-C₇ alkylene group or a C₂-C₇ alkylene group having one or more branches (the number of the carbon atoms including the carbon atoms in the branch(es) is one or more and seven or less). Examples include methylene, ethylene, propylene, and butylene. More specific examples include -CH₂-, -(CH₂)₂-, -(CH₂)₃-, - CH(CH₃)-, -(CH₂)₃-, -(CH(CH₃))₂-, -(CH₂)₂-CH(CH₃)-, -(CH₂)₃-CH(CH₃)-, -(CH₂)₂-CH(C₂H₅)-, -(CH₂)₆-, -(CH₂)₂-C(C₂H₅)₂-, and -(CH₂)₃C(CH₃)₂CH₂-.

"C₂-C₇ alkenylene" is a linear or branched alkenylene group having two to seven carbon atoms, and having one or more double bonds in a chain. The alkenylene is, for example, a divalent group having a double bond, obtained by removing two to five of the hydrogen atoms at the coadjacent carbon atoms from the alkylene group.

Herein, an alkylene group and an alkenylene group may have any one or more substituents. Examples of such a substituent include, but are not limited to, alkoxy, halogen atom (which may be any one of a fluorine atom, chlorine atom, bromine atom, and iodine atom), amino, mono- or di-substituted amino, substituted silyl, acyl, or aryl. When two or more substituents are present, the substituents may be the same or different.

In addition, when a functional group is herein defined as a group that "may have a substituent", the kind of the substituent, the position of substitution, and the number of substituents are not particularly limited. When two or more substituents are present, the substituents may be the same or different. Examples of the substituent include, but are not limited to, alkyl, alkoxy, hydroxyl, carboxyl, halogen atom, sulfo, amino, alkoxy carbonyl, and oxo. These substituents may further contain a substituent.

### <Linear Polymer>

The linear polymer that forms a hydrogel through a cross-linking reaction with a multibranched polymer contains two or more nucleophilic functional groups or electrophilic functional groups in at least one of a side chain and a terminal. For cross-linking, the linear polymer has a nucleophilic functional group when the multibranched polymer has an electrophilic functional group, and the linear polymer has an electrophilic functional group when the multibranched polymer has a nucleophilic functional group. The details about the electrophilic functional group and the nucleophilic functional group are the same as the details about the above-described multibranched polymer.

The linear polymer is a protein, peptide, or polysaccharide. The protein is not particularly limited as long as the protein does not adversely affect a cell, and can be suitably selected according to the purpose. Examples include collagen, fibrin, and albumin. In particular, gelatin is preferably used as the linear polymer. In addition, a peptide is two or more amino acid residues polymerized via a peptide bond (amide bond). The peptide may be any one of a dipeptide, tripeptide, oligopeptide (containing approximately 10 amino acids), and polypeptide (containing tens to hundreds of amino acids), and preferably does not gelate at normal temperature under normal pressure. Furthermore, the polysaccharide is not particularly limited, and can be suitably selected. Examples include cellulose, chitin, chitosan, hyaluronic acid, alginic acid, starch, and pectin. These linear polymers may be used singly or in combination of two or more kinds thereof. A linear polymer containing two or more nucleophilic functional groups or electrophilic functional groups in at least one of a side chain and a terminal is illustrated below with reference to a gelatin having a thiol group in a side chain.

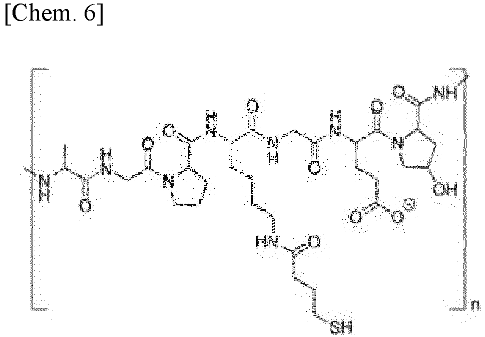

### <Cell>

The species or the like of the cell is not particularly limited, and can be suitably selected according to the purpose. Any species of cell can be used, taxonomically irrespective of whether the cell is, for example, a eukaryotic cell, prokaryotic cell, multicellular organism cell, or unicellular organism cell.

Examples of the eukaryotic cell include animal cells, insect cells, plant cells, and fungi. These may be used singly or in combination of two or more species thereof. Among these, animal cells are preferable. In a case where the cells form a cell aggregate, adhesive cells adherent to each other, and having such cell adhesiveness as to be not isolated, unless physicochemically treated, are more preferable.

The adhesive cell is not particularly limited, and can be suitably selected according to the purpose. Examples include differentiated cells and undifferentiated cells.

Examples of the differentiated cell include: a hepatocyte that is a parenchymal cell of liver; stellate cell; Kupffer cell; vascular endothelial cell; endothelial cell such as sinusoidal endothelial cell or corneal endothelial cell; fibroblast; osteoblast; osteoclast; periodontiumderived cell; epidermal cell such as epidermal keratinocyte; tracheal epithelial cell; gastrointestinal epithelial cell; uterocervical epithelial cell; epithelial cell such as corneal epithelial cell; lactocyte; pericyte; myocyte such as smooth muscle myocyte or cardiomyocyte; nephrocyte; pancreas islet cell; neurocyte such as peripheral neurocyte or optic nerve cell; chondrocyte; and osteocyte. The adhesive cell may be a primary cell collected directly from a tissue or an organ, or may be a cell obtained by subjecting the primary cell to a passage(s).

The undifferentiated cell is not particularly limited, and can be suitably selected according to the purpose. Examples include: an embryonic stem cell that is an undifferentiated cell; a pluripotent stem cell such as a mesenchymal stem cell having pluripotency; a unipotent stem cell such as an endothelial precursor cell having unipotency; and an iPS cell.

### Examples of the prokaryotic cell include eubacteria and archaea.

Specific examples of the cell include a normal human dermal fibroblast. As the normal human dermal fibroblast, a commercially available product can be used. Examples of the commercially available product include a product under the tradename CC2507 (manufactured by Lonza Group AG).

### <Cell-containing Gel Particles>

A cell-containing fine gel particle is formed in vitro, and accordingly, a culture container, culture medium, and known material for maintaining the functional expression, cell survival, and the like are used suitably according to the usage. The cell-containing gel particle contains one or more cells, and is in spherical or ellipsoidal shape. The gel particle may contain a plurality of species of cells, and may be in contact with a base plate such as a culture container. The cell density is not particularly limited, and the cells may be directly bound to each other. The hydrogel present around the cell is a gel obtained through a cross-end coupling reaction between a multibranched polymer and a linear polymer. The gel concentration (the mass% concentration (w/w) of the gel molecules in the hydrogel) is preferably 0.6% to 8%.

The diameter of the cell-containing gel particle can be suitably set according to the species of the cell and the cell-culturing state, and is preferably in the range of from 60 µm to 500 µm owing to the easiness of observing the cell contained in the hydrogel. Here, the diameter refers to the average of the long axis and short axis of a gel particle in a micrograph of a cell-containing gel particle, as observed under a microscope.

The hydrogel 50 that encapsulates a cell 51 may be supplemented with an extracellular matrix, if desired. The extracellular matrix is not particularly limited, and can be suitably selected according to the purpose. Examples include: extracellular matrix proteins and sugar proteins, such as collagen, laminin, fibronectin, elastin, fibrin, proteoglycan, hyaluronic acid, heparan sulfate proteoglycan, and chondroitin sulfate proteoglycan; and proliferators and growth factors, such as hepatocyte proliferators, fibroblast proliferators, and nerve growth factors, depending on the cell. These may be used singly or in combination of two or more kinds thereof. It is known that such an extracellular matrix promotes the adhesion, proliferation, or differentiation of a cell. Contained in a hydrogel 50, the extracellular matrix can function as a trigger that changes the form of a cell in the gel particle formed.

### <Method for Producing Cell-containing Gel Particles>

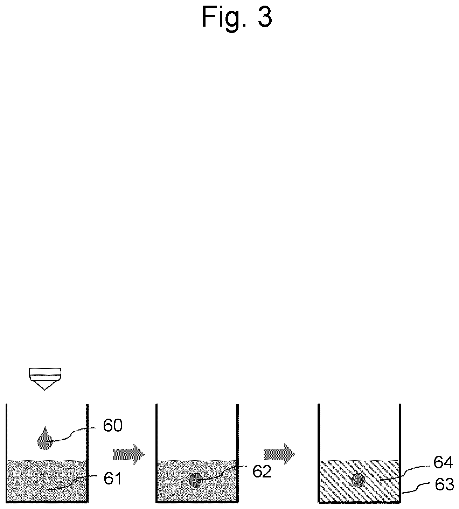

A method for producing the above-described cell-containing gel particle will be described with reference to FIG. 3. A cell-containing gel particle 62 in the present embodiment can be produced by mixing the following: a droplet of a first solution 60 containing a multibranched polymer and a cell; a second solution 61 containing no cell, containing a linear polymer, and placed in a culture container 63. Alternatively, the cell may be contained not in the first solution but in the second solution, and the droplet of the second solution may be mixed with the first solution containing no cell.

### <Culture Container>

The culture container 63 is composed of a base material serving as a base or a support for forming and maintaining a cell-containing gel particle. Examples include a container made of resin, glass, metal, or the like. The shape of a usable base material is planar, perforated, mesh-like, uneven, or honeycomb, and may be suitably selected according to the usage. A multiwell culture plate or insert that has high optical transparency, and emits neither autofluorescence nor light is preferably used.

In the example in FIG. 3, the cell-containing gel particle 62 formed through cross-linking between the first solution 60 and the second solution 61 is present in a culture medium 64 contained in a culture container 63, and in this state, the cell can be cultured. As illustrated in FIG. 3, in a cell culture performed in a state where the cell-containing gel particle 62 is surrounded by the culture medium 64, even one cell can be cultured. The cell that survives and spreads in the cell-containing gel particle 62 can be observed. In a case where a neurocyte is used, it is characteristic of the neurite to stay in the cell-containing gel particle 62. In the example in FIG. 3, the top of the culture container 63 is opened for the culture medium 64 or a drug to be taken in and out. A closed system usable for a circumfusion culture or a chip type of container having a micro flow path may be used.

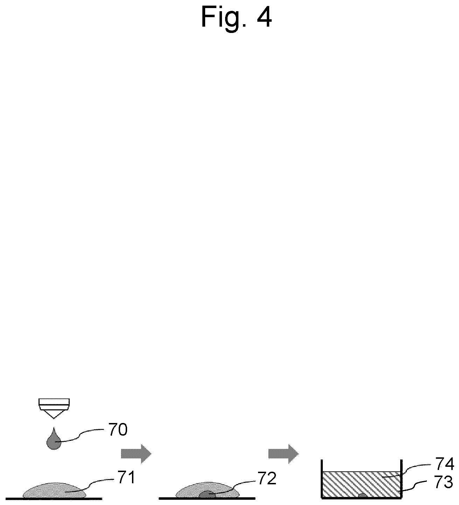

As illustrated in FIG. 4, it is possible in another embodiment that a droplet of a first solution 70 containing a cell is dropped onto a second solution 71 on a base plate, a cell-containing gel particle 72 is formed through a cross-linking reaction, this cell-containing gel particle 72 is allowed to adhere to a culture container 73, and retained, and the cell is cultured in the culture container 73 full of a culture medium 74. Performing a cell culture in a state where the cell-containing gel particle 72 is in contact with the culture container 73 has advantages in that a floating cell can be cultured under contact conditions, and that microscopic observation is easier.

### <Culture Medium>

A culture medium contained in a culture container is a solution that contains a component for maintaining the shape of a cell-containing gel particle, and maintaining the survival of a cell, prevents drying, and conditions the external environment such as an osmotic pressure. The culture medium is not particularly limited, and can be suitably selected from known culture media according to the usage.

Examples of the culture medium include: culture media classified according to the composition, such as a natural medium, semisynthetic medium, and synthetic medium; culture media classified according to the shape, such as a semisolid medium, liquid culture medium, and powdery medium (hereinafter referred to as a "powder medium" in some cases). These may be used singly or in combination of two or more kinds thereof. Any cell derived from an animal can be used as long as the cell is a culture medium usable to culture an animal cell.

The culture medium to be used to culture an animal cell is not particularly limited, and can be suitably selected according to the purpose. Examples include Dulbecco's Modified Eagles's medium (D-MEM), Ham's F12 medium (Ham's Nutrient Mixture F12), D-MEM/F12 medium, McCoy's 5A medium, Eagles's MEM medium (Eagles's Minimum Essential Medium; EMEM), αMEM medium (alpha Modified Eagles's Minimum Essential Medium; αMEM), MEM medium (Minimum Essential Medium), RPMI1640 medium, Iscove's Modified Dulbecco's medium (IMDM), MCDB131 medium, William's medium E, IPL41 medium, Fischer's medium, StemPro-34 (manufactured by Invitrogen), X-VIVO 10 (manufactured by Cambrex Corporation), X-VIVO 15 (manufactured by Cambrex Corporation), HPGM (manufactured by Cambrex Corporation), StemSpan H3000 (manufactured by Stem Cell Technology Inc.), StemSpan SFEM (manufactured by Stem Cell Technology Inc.), Stemline II (manufactured by Sigma-Aldrich), QBSF-60 (manufactured by Quality Biological Inc.), StemPro hESC SFM (manufactured by Invitrogen), Essential 8 (registered trademark) medium (manufactured by Gibco), Stemfit AK02N (manufactured by Ajinomoto Co., Inc.), Stemfit Basic 02 (manufactured by Ajinomoto Co., Inc.), mTeSR 1 or 2 medium (manufactured by Stem Cell Technology Inc.), ReproFF or ReproFF2 (manufactured by Reprocell Inc.), PSGro hESC/iPSC medium (manufactured by System Biosciences, LLC), NutriStem (registered trademark) medium (manufactured by Biological Industries Ltd.), CSTI-7 medium (manufactured by Cell Science & Technology Institute, Inc.), MesenPRO RS medium (manufactured by Gibco), MF-Medium (registered trademark) mesenchymal stem cell growth medium (manufactured by Toyobo Co., Ltd.), Sf-900II (manufactured by Invitrogen), and Opti-Pro (manufactured by Invitrogen). These may be used singly or in combination of two or more kinds thereof.

The carbon dioxide concentration of a culture medium is not particularly limited, can be suitably selected according to the purpose, and is preferably 2% or more and 5% or less. Having the carbon dioxide concentration at 2% or more and 5% or less enables a cell to be cultured suitably.

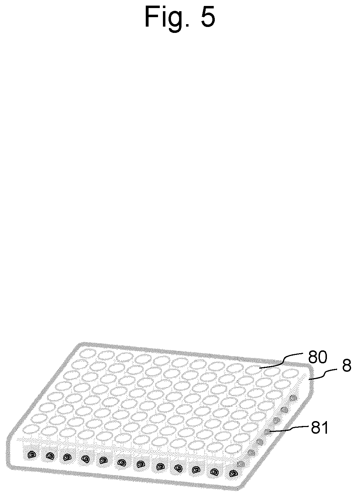

FIG. 5 illustrates one embodiment of a cell-evaluating plate according to the present invention. In a cell-evaluating plate 8 in FIG. 5, a base plate has many wells 80, and each well 80 contains a cell-containing gel particle 81. The number of wells is not particularly limited. In addition, the individual cell-containing gel particles 81 may have the same composition or different compositions in terms of the species or the like of the cell or the hydrogel. Such a cell-evaluating plate 8 can be suitably used for high-through put screening.

### EXAMPLES

Below, the present invention will be further described specifically with reference to Examples and Comparative Examples. However, the technical scope of the present invention is not limited to these Examples.

### (Example 1)

### (1) Preparation of First Solution

In 1 ml of phosphate buffered saline (manufactured by Life Technologies Corp.; hereinafter also referred to as PBS (-)), 0.02 g of Tetra-PEG-maleimidyl (SUNBRIGHT PTE-100MA, manufactured by Yuka Sangyo Co., Ltd.) was dissolved to prepare a first solution containing 2% Tetra-PEG-maleimidyl.

### (2) Preparation of Second Solution

To 0.05 g of gelatin-SH (thiol functionalized gelatin, manufactured by Sigma-Aldrich), 1 ml of PBS (-) was added, and the gelatin was dissolved at 37°C overnight to prepare a second solution containing 5% gelatin-SH.

### (3) Production of Hydrogel

Into a 96 well plate, 10 µL of the second solution prepared in (2) was dropped, and then, the second solution dropped was stirred and mixed with 10 µL of the first solution produced in (1), to produce a hydrogel.

### (4) Seeding of Neurocyte

Freeze-preserved glutamatergic neurocytes in a vial were thawed in a warm bath at 37°C, and then transferred to one 15 mL centrifuge tube, and 5 mL of DMEM was added to suspend the neurocytes. From the cell suspension, 100 µL thereof was taken out into an Eppendorf tube, and the sample was sucked into a cassette. The number of cells was measured using a cell counter, and the number of cells in the liquid was determined. The cell suspension in the 15 mL centrifuge tube was centrifuged (at 200 ×g for 3 minutes), an aspirator was used to remove the supernatant, and then, the cell suspension was adjusted to a cell concentration of 1 × 10⁶ cells/mL with PBS (-). In the hydrogel produced in (3), the cell suspension was seeded so as to contain 1 × 10⁴ cells, whereby a cell culture body was produced.

### (5) Observation of Cell

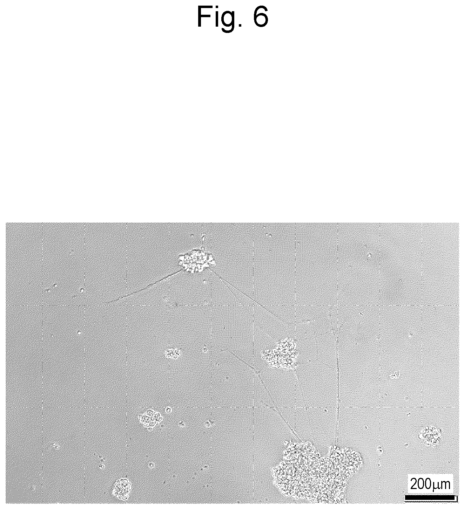

With the cell culture body produced, the form of the cells on the surface of the hydrogel after seven days of culture was evaluated according to whether the cells spread. The form of the cells in the hydrogel was observed under a microscope (CKX41, manufactured by Olympus Corporation). The results are illustrated in FIG. 6. As illustrated in FIG. 6, the spreading of the neurocytes on the surface of the hydrogel was verified.

### (Example 2)

### (1) Preparation of First Solution

In 1 ml of phosphate buffered saline (manufactured by Life Technologies Corp.; hereinafter also referred to as PBS (-)), 0.02 g of Tetra-PEG-maleimidyl (SUNBRIGHT PTE-100MA, manufactured by Yuka Sangyo Co., Ltd.) was dissolved to prepare a first solution containing 2% Tetra-PEG-maleimidyl.

### (2) Preparation of Second Solution

To 0.05 g of gelatin-SH (thiol functionalized gelatin, manufactured by Sigma-Aldrich), 1 ml of PBS (-) was added, and the gelatin was dissolved at 37°C overnight to prepare a second solution containing 5% gelatin-SH.

### (3) Culture of Human Umbilical Vein Endothelial Cell (hereinafter referred to as "HUVEC" or "HUVEC cell")

HUVEC cells were cultured using an endothelial-cell basal medium (culture system containing EBMTM-2 Basal Medium (CC-3156) and EGMTM-2 SingleQuotsTM Supplements (CC-4176), manufactured by Lonza Group AG) in a 100 mm dish in a 37°C and 5 vol% CO₂ environment in an incubator (KM-CC17RU2, manufactured by Panasonic Corporation, in a 37°C and 5 vol% CO₂ environment).

### (4) Production of First Solution Containing HUVEC Cells

To a dish, 5 mL of PBS (-) was added, and the PBS (-) was removed by suction using an aspirator to wash the surface. After the washing with PBS (-), 2 mL of a 0.05% trypsin-0.05% EDTA solution (manufactured by Life Technologies Corp.) was added to the dish, the resulting mixture was heated in an incubator for 5 minutes, and the cells were peeled off from the dish. Under a phase-contrast microscope (device name: CKX41, manufactured by Olympus Corporation), the cells peeled off were verified. Then, 2 mL of FBS-containing DMEM was added to the dish to deactivate the trypsin. The cell suspension in the dish was transferred into one 15 ml centrifuge tube, and centrifuged (H-19FM, manufactured by KOKUSAN; at 200 ×g for 3 minutes). An aspirator was used to remove the supernatant. After the removal, 2 mL of FBS-containing DMEM was added to the centrifuge tube. The resulting mixture was gently pipetted, and the cells were dispersed to obtain a cell suspension. From the cell suspension, 100 µL thereof was taken out into an Eppendorf tube, and the sample was sucked into a cassette (Via1-Cassette, manufactured by ChemoMetec A/S). The number of cells was measured using a cell counter (NucleoCounter NC-3000, manufactured by ChemoMetec A/S), and the number of cells in the liquid was determined. Part of the cell suspension was transferred into a 15 ml centrifuge tube, and centrifuged (at 200 ×g for 3 minutes). A pipette was used to remove the supernatant. The first solution prepared in (1) was added to obtain a cell-containing first solution containing 2% Tetra-PEG-maleimidyl, and composed of a cell suspension having a cell concentration of 5 × 10⁶ cells/mL.

### (5) Production of Cell Culture Body

Into a 35 mm dish (Model No.: 3000-035, manufactured by AGC Technoglass Co., Ltd.), 3 µL of the second solution prepared in (2) was dropped, and then, the second solution dropped was stirred and mixed with 3 µL of the cell-containing first solution produced in (4), to produce a cell culture body including a hydrogel and cells encapsulated in the hydrogel.

### (6) Observation of Cell

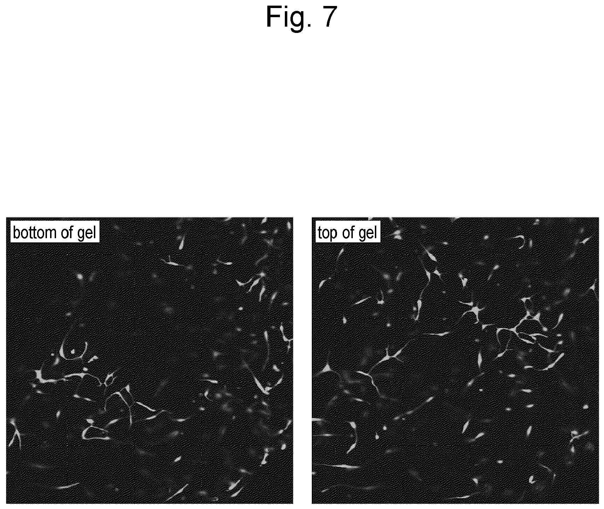

With the cell culture body produced, the form of the cells in the hydrogel after seven days of culture was evaluated according to whether the cells spread. Calcein AM (-Cellstain (registered trademark)-Calcein-AM solution, manufactured by Fujifilm Wako Pure Chemical Corporation) was added to the culture medium in the 35 mm dish, and a culture was again performed in a 37°C and 5 vol% CO₂ environment in the incubator (as above-described) for one hour. After the one-hour culture, the form of the cells in the hydrogel was observed under a confocal microscope. The results are illustrated in FIG. 7. As illustrated in FIG. 7, the spreading of the HUVEC cells in the hydrogel was verified.

### (Example 3)

The basic procedures were in accordance with Example 2. However, the present Example is different from Example 2 in that two or more kinds of gel precursors were formed at mixing ratios made different from the first solution, that neurocytes were used instead of HUVEC cells, and the cell culture body was produced by inkjet.

### (1) Preparation of First Solution

In 1 ml of PBS (-), 0.02 g of Tetra-PEG-maleimidyl was dissolved. Furthermore, using the second solution produced in Example 2, a first solution containing 2% Tetra-PEG-maleimidyl and 0.1% Thiol Gelatin was prepared.

### (2) Production of First Solution Containing Neurocytes

Freeze-preserved glutamatergic neurocytes in a vial were thawed in a warm bath at 37°C, and then transferred to one 15 mL centrifuge tube, and 5 mL of DMEM was added to suspend the neurocytes. From the cell suspension, 100 µL thereof was taken out into an Eppendorf tube, and the sample was sucked into a cassette. The number of cells was measured using a cell counter, and the number of cells in the liquid was determined. The cell suspension in the 15 mL centrifuge tube was centrifuged (at 200 ×g for 3 minutes). An aspirator was used to remove the supernatant. The first solution prepared in (1) was added to obtain a cell-containing first solution containing 1% Tetra-PEG-maleimidyl and 0.1% gelatin-SH, and composed of a cell suspension having a cell concentration of 1 × 10⁷ cells/mL.

### (3) Production of Cell Culture Body

The second solution in Example 2 was applied to an 18 mm square cover glass (No.1 Thickness 0.13 to 0.17 mm, manufactured by Matsunami Glass Ind., Ltd.). The cell-containing first solution prepared in (2) was fed into the liquid chamber of an ink jet head, from which droplets were dropped one by one onto the cover glass. The cell-containing first solution was landed onto the second solution, and the two liquids reacted to produce a cell culture body. After the gel was formed, the cover glass was transferred to a 35 mm dish, to which a culture medium was added, followed by verifying the formation of a gel under a microscope. The dish was placed in a 37°C and 5% CO₂ environment in an incubator, and the cells were cultured for 14 days.

### (4) Immunofluorescent Staining

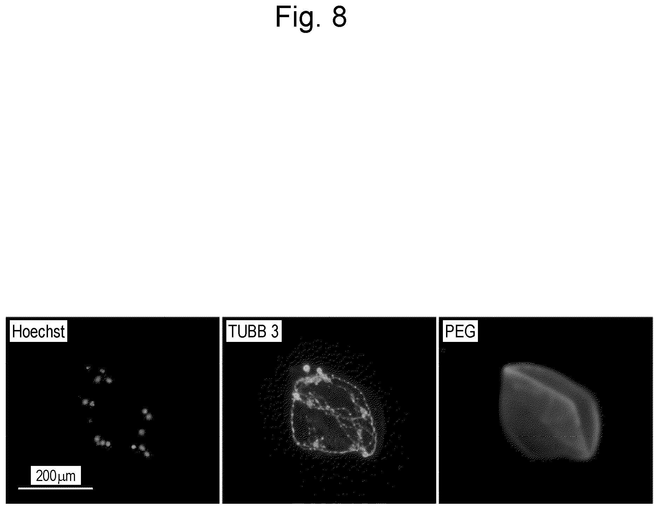

After the supernatant was removed, PBS (-) was added for washing (hereinafter referred to as "PBS washing"). After the supernatant was removed, 4% paraformaldehyde (hereinafter referred to as "4% PFA") was added, and the resulting mixture was left to stand for 20 minutes to fix the cells. After the supernatant was removed, PBS washing was repeated three times. After the supernatant was removed, 0.1% Triton was added for a 10-minute cell membrane permeation treatment. After the PBS washing was repeated three times, the cells were subjected to blocking with 1% BSA for one hour. A β3 tubulin antibody (β3-Tubulin (D71G9) XP Rabbit mAb #5568, manufactured by Cell Signaling Technology, Inc.; hereinafter referred to as "TUBB3") and a PEG antibody (Anti-PEG antibody-BSA and Azidefree by Rat monoclonal, Abcam plc) were diluted with 1% BSA. After the blocking, the supernatant was removed, the primary antibody solution diluted was added, and the resulting mixture was incubated at 4°C overnight. The next day, PBS washing was repeated three times, the secondary antibody solution diluted with a 1% BSA solution was added, and the resulting mixture was incubated for one hour. The following operations were performed under a light shield of aluminum foil or the like. After the supernatant was removed, a Hoechst solution diluted with PBS was added, and the resulting mixture was subjected to nuclear stain for 5 minutes. PBS washing was repeated twice, and a mounting agent was added dropwise. Observation under a fluorescence microscope (Carl Zeiss AG) verified that the cells in the hydrogel on culture day 14 expressed TUBB3 that is a marker of a neurocyte (FIG. 8). In addition, the neurites were bent along the outline of the gel, thus allowing the observation of how the neurites remained in the gel.

### (Example 4)

The basic procedures were in accordance with Example 3. However, the present Example is different from Example 3 in that HUVEC cells and HepG2 cells (human liver cancerderived cell strains) were used, and that the cell culture body was laminated by inkjet.

### (1) Culture of HepG2 Cell

In an incubator, HepG2 cells in a 100 mm dish were cultured for 72 hours, using Dulbecco's Modified Eagle Medium (tradename: DMEM (1X), manufactured by Thermo Fisher Scientific Inc.; hereinafter referred to as "DMEM") containing 10 mass% fetal bovine serum (hereinafter referred to as "FBS") and 1 mass% antibiotic (Antibiotic-Antimycoti c Mixed Stock Solution (100×), manufactured by Nacalai Tesque, Inc.).

### (2) Production of First Solution Containing HepG2 Cells

The cell suspension having HepG2 cells dispersed therein was obtained in the same manner as the HUVEC cells. The first solution prepared was added to obtain a HepG2-cell-containing first solution containing 2% Tetra-PEG-maleimidyl, and composed of a cell suspension having a cell concentration of 1 × 10⁷ cells/mL.

### (3) Production of First Solution Containing HUVEC Cells

In the same manner as in Example 2, a HUVEC-cell-containing first solution composed of a cell suspension having a cell concentration of 1 × 10⁷ cells/mL was obtained.

### (4) Production of Cell Culture Body

From an ink jet head fed with the HepG2-cell-containing first solution, droplets were dropped one by one at 100 µm pitches in a 10 mm × 20 mm region on an 18 mm square cover glass. Then, the resulting object was immersed in a dish containing the second solution, and the HepG2-cell-containing first solution in droplet form was impregnated with the second solution to form a cell culture body.

Subsequently, from an inkjet head fed with the HUVEC-cell-containing first solution, droplets were dropped one by one at 100 µm pitches in a 5 mm × 10 mm region on the cell culture body. Then, the HUVEC-cell-containing first solution in droplet form was impregnated with the second solution to form a cell culture body as a second layer.

The same operation with the inkjet head was repeated to form cell culture bodies as a third layer (HepG2 cells) and a fourth layer (HUVEC cells), finally producing a three-dimensional cell culture body composed of a four-layer cell-containing hydrogel.

Finally, the three-dimensional cell culture body was transferred into a 35 mm dish containing: 1 mL of DMEM containing 10 mass% FBS and 1 mass% antibiotic; and 1 mL of an endothelial cell basal culture medium. The dish was placed in a 37°C and 5 vol% CO₂ environment in an incubator (as above-described).

### (5) Immunofluorescent Staining

After the supernatant was removed, PBS (-) was added for washing. After the supernatant was removed, 4% PFA was added, and the resulting mixture was left to stand for 20 minutes to fix the cells. After the supernatant was removed, PBS washing was repeated three times. After the supernatant was removed, 0.1% Triton was added for 10-minute cell membrane permeation treatment. After the PBS washing was repeated three times, the cells were subjected to blocking with 1% BSA for one hour. An albumin antibody (Anti-Albumin antibody, manufactured by Abcam plc) and a CD31 antibody (Anti-CD31 antibody, Abcam plc) were diluted with 1% BSA. After the blocking, the supernatant was removed, the primary antibody solution diluted was added, and the resulting mixture was incubated at 4°C overnight. The next day, PBS washing was repeated three times, the secondary antibody solution diluted with a 1% BSA solution was added, and the resulting mixture was incubated for one hour. The following operations were performed under a light shield of aluminium foil or the like. After the supernatant was removed, a Hoechst solution diluted with PBS was added, and the resulting mixture was subjected to nuclear stain for 5 minutes. PBS washing was repeated twice, and a mounting agent was added dropwise. Observation under a confocal microscope (FV10, manufactured by Olympus Corporation) verified that the cells in the hydrogel on culture day 7 expressed albumin that is a marker of a HepG2 cell and CD31 that is a marker of a HUVEC cell, and that the cells spread. The observation also verified the formation of a layered structure having HepG2 cells in the first and third layers, and HUVEC cells in the second and fourth layers.

### (Example 5)

The basic procedures were in accordance with Example 3. However, the present Example is different from Example 3 in that HUVEC cells were used, and that the cell culture body was laminated by ink jet.

### (1) Preparation of Cell-free First Solution

In 1 ml of phosphate buffered saline (manufactured by Life Technologies Corp.; hereinafter also referred to as PBS (-)), 0.02 g of Tetra-PEG-maleimidyl (SUNBRIGHT PTE-100MA, manufactured by Yuka Sangyo Co., Ltd.) was dissolved to prepare a cell-free first solution containing 2% Tetra-PEG-maleimidyl.

### (2) Production of First Solution Containing HUVEC Cells

A first solution containing 1% Tetra-PEG-maleimidyl was prepared, and a HUVEC-cell-containing first solution composed of a cell suspension having a cell concentration of 1 × 10⁷ cells/mL was obtained.

### (3) Production of Cell Culture Body

From an inkjet head fed with a cell-free first solution, droplets were dropped one by one at 100 µm pitches in a 5 mm × 10 mm region on an 18 mm square cover glass. Then, the cover glass was placed in a dish containing the second solution prepared in Example 2, and the cell-free first solution in droplet form was impregnated with the second solution to form a hydrogel.

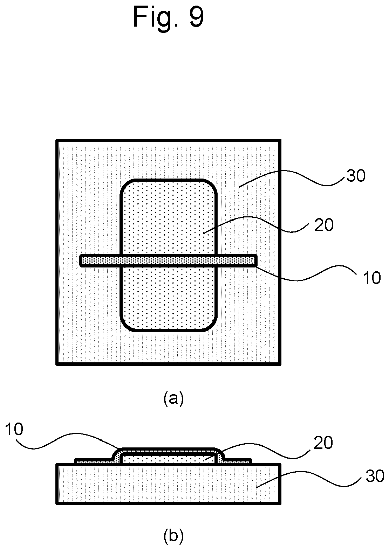

Subsequently, a step of forming a gel laminate was performed, in which, from an inkjet head fed with the HUVEC-cell-containing first solution, droplets were dropped one by one at 100 µm pitches so as to traverse the hydrogel, as illustrated in FIG. 9. Thus, a laminate was produced, in which the hydrogel 20 was arranged on a cover glass 30, and a linear cell culture body 10 having a length of 15 mm was formed so as to traverse the hydrogel 20.

### (4) Immunofluorescent Staining

After the supernatant was removed, PBS (-) was added for washing. After the supernatant was removed, 4% PFA was added, and the resulting mixture was left to stand for 20 minutes to fix the cells. After the supernatant was removed, PBS washing was repeated three times. After the supernatant was removed, 0.1% Triton was added for a 10-minute cell membrane permeation treatment. After the PBS washing was repeated three times, the cells were subjected to blocking with 1% BSA for one hour. A CD31 antibody (Anti-CD31 antibody, manufactured by Abcam plc) was diluted with 1% BSA. After the blocking, the supernatant was removed, the primary antibody solution diluted was added, and the resulting mixture was incubated at 4°C overnight. The next day, PBS washing was repeated three times, the secondary antibody solution diluted with a 1% BSA solution was added, and the resulting mixture was incubated for one hour. The following operations were performed under a light shield of aluminum foil or the like. After the supernatant was removed, a Hoechst solution diluted with PBS was added, and the resulting mixture was subjected to nuclear stain for 5 minutes. PBS washing was repeated twice, and a mounting agent was added dropwise. Observation under a confocal microscope (FV10, manufactured by Olympus Corporation) verified that the cells in the hydrogel on culture day 7 expressed CD31 that is a marker of a HUVEC cell, and that the cells spread.

### (Example 6)

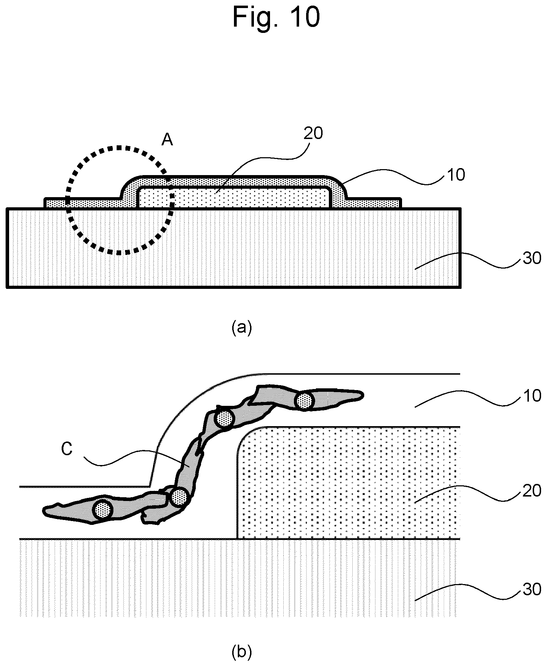

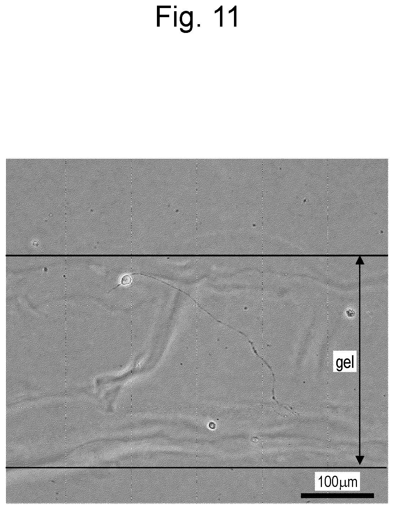

The basic procedures were in accordance with Example 5. However, the present Example is different from Example 5 in that neurocytes used in Example 3 were used. Cell-containing ink was dropped by ink jet, whereby, as illustrated in FIG. 10 (a), a laminate was produced, in which a hydrogel 20 was arranged on a cover glass 30, and a linear cell culture body 10 was formed so as to traverse the hydrogel 20. In this laminate, cells C spread in the cell culture body 10, as illustrated in FIG. 10 (b) that is a schematic enlarged view of the portion A in FIG. 10 (a). FIG. 11 is an image of the linear cell culture body 10 as observed under a microscope. The result in FIG. 11 has verified that, when a linear cell culture body was produced by ink jet, the cells spread along the whole line without depending on the shape of the droplet discharged.

### (Comparative Example 1)

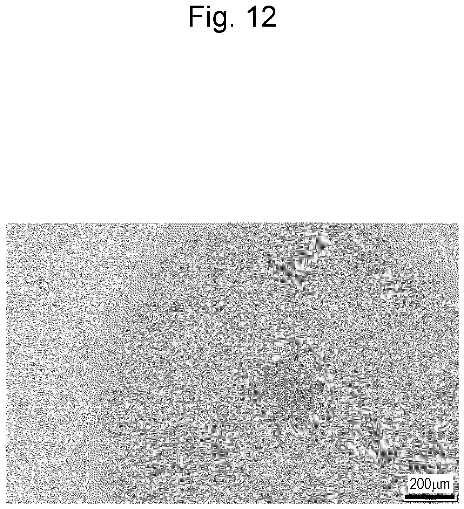

Using Tetra-PEG-maleimidyl as the first solution, and using Tetra-PEG-SH as the second solution, a hydrogel was produced and observed in the same manner as in Example 1. As illustrated in FIG. 12, the neurocytes remained round without spreading, that is, almost no cell spread.

### (Comparative Example 2)

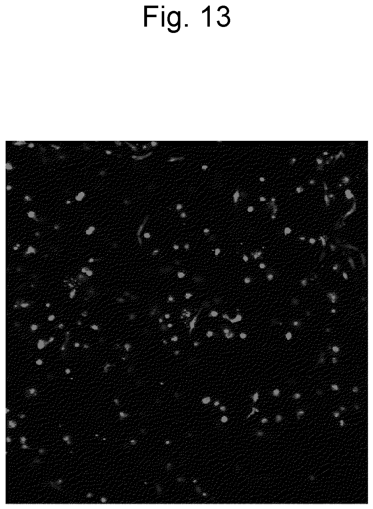

The production of a cell culture body and the observation of the spreading of cells were performed in the same manner as in Example 2 except that a gel used as a hydrogel covering the cells was produced by mixing the following: Tetra-PEG-maleimidyl and Tetra-PEG-SH (SUNBRIGHT PTE-100SH, manufactured by Yuka Sangyo Co., Ltd.) at a mass ratio of 1% with respect to phosphate buffered saline; thrombin (Thrombin from bovine Plasma, manufactured by Sigma-Aldrich) at 20 U/mL; and fibrinogen (Fibrinogen from bovine plasma, manufactured by Sigma-Aldrich) at a mass ratio of 1%. The results are illustrated in FIG. 13. FIG. 13 has verified that the form of a HUVEC cell after incubation was round, and that the cells spread, and suggests that it is difficult to spread almost all cells, and to culture the cells for a long time in a hydrogel.

### (Comparative Example 3)

In Comparative Example 3, the production of a cell culture body and the observation of the spreading of cells were performed in the same manner as in Example 2 except that a calcium alginate gel was formed by mixing, at a mass ratio of 1:1, the following: a sodium alginate solution in which sodium alginate (SKAT ONE, manufactured by Kimica Corporation) and gelatin-SH were dissolved at a mass ratio of 1% and a mass ratio of 2% respectively in phosphate buffered saline (manufactured by Life Technologies Corp.; hereinafter referred to as PBS (-)); and a calcium chloride solution in which calcium chloride dihydrate (manufactured by Wako Pure Chemical Industries, Ltd.) was dissolved at 100 mmol/L in distilled water. As a result, the form of the HUVEC cell after incubation remained round without spreading. Furthermore, it has been verified that the calcium alginate gel gradually collapsed as the dish was shaken, and that the gel lacked stability as a scaffold material for culture.

### (Comparative Example 4)

Using Tetra-PEG-maleimidyl and Matrigel as the first solution, and using Tetra-PEG-SH as the second solution, a cell culture body was produced and observed in the same manner as in Example 3. The neurocytes remained round without spreading, that is, almost no cell spread.

A cell culture body formed by the method in each of Examples and Comparative Examples was evaluated by the method described below, and the results are tabulated in Table 1.

### (Evaluation of Form of Cell after Long-time Culture in Gel)

According to the form of the cell after culture day 7, the spreading of 50% or more of the cells was rated A, and no spreading of the cells was rated X. In a case where a pseudopod of the cell was found, it was judged that the cells spread.

**[Table 1]**

| | Example 1 | Example 2 | Example 3 | Example 4 | Example 5 |
|---|---|---|---|---|---|
| Composition of Gel | PEG-Maleimidyl Gelatin-SH | PEG-Maleimidyl Gelatin-SH | PEG-Maleimidyl Gelatin-SH | PEG-Maleimidyl Gelatin-SH | PEG-Maleimidyl Gelatin-SH |
| Method for Producing Gel | Manual Operation | Manual Operation | Ink jet | Ink jet Lamination | Ink jet Lamination |
| Spreading of Cells | A | A | A | A | A |

| | Example 6 | Comparative Example 1 | Comparative Example 2 | Comparative Example 3 | Comparative Example 4 |
|---|---|---|---|---|---|
| Composition of Gel | PEG-Maleimidyl Gelatin-SH | PEG-Maleimidyl PEG-SH | PEG-Maleimidyl PEG-SH Fibrin | Alginic Acid Gelatin-SH | PEG-Maleimidyl PEG-SH Matrigel |
| Method for Producing Gel | Ink jet Lamination | Manual Operation | Manual Operation | Manual Operation | Ink jet |
| Spreading of Cells | A | X | X | X | X |

The results in Table 1 have verified that the cells can spread on the surface of the hydrogel in Example 1, and that the cells do not spread on the surface of the usual Tetra-PEG gel in Comparative Example 1. It has been verified that the cell culture body in Example 2 is a cell culture body capable of efficiently spreading HUVEC cells, compared with the usual Tetra-PEG gel (Comparative Example 2) supplemented with a guest, and compared with the alginic acid gel (Comparative Example 3). In addition, Example 3 and Comparative Example 4 have verified that even a cell culture body produced by inkjet can afford the same results. That is, it has successfully been verified that forming a hydrogel through cross-linking between a polymer material fast in gelation and a water-soluble protein having cell adhesiveness, in accordance with the present invention, allows producing a cell culture body having both gel shapeability and cell adhesiveness, and allows a long-time cell culture.

### (Example 7) Production of Cell-containing Gel Particles Containing 3T3 Cell (Adhesive Cell)

### (1) Preparation of First Solution

In 1 ml of phosphate buffered saline (manufactured by Life Technologies Corp.; hereinafter also referred to as PBS (-)), 0.02 g of Tetra-PEG-maleimidyl (tradename: SUNBRIGHT PTE-100MA, manufactured by Yuka Sangyo Co., Ltd.) was dissolved to prepare a first solution containing 2% Tetra-PEG-maleimidyl.

### (2) Preparation of Second Solution

To 0.05 g of Thiol Gelatin (tradename: Thiol functionalized gelatin, manufactured by Sigma-Aldrich), 1 ml of PBS (-) was added, and the gelatin was dissolved at 37°C overnight to prepare a second solution containing 5% Thiol Gelatin.

### (3) Culture of Cell

In an incubator (tradename: KM-CC17RU2, manufactured by Panasonic Corporation; in a 37°C and 5% CO₂ environment), 3T3/NIH cells (JCRB Cell Bank, hereinafter referred to as "3T3") were cultured in a 100 mm dish for 72 hours, using Dulbecco's Modified Eagle Medium (tradename: DMEM (1X), manufactured by Life technologies Corp., hereinafter referred to as "DMEM") containing 10% neonatal calf serum (tradename: Newborn Calf Serum, manufactured by Sigma-Aldrich; hereinafter referred to as "NCS").

### (4) Production of Cell Suspension

To a dish, 5 mL of PBS (-) was added, and the PBS (-) was removed by suction using an aspirator to wash the surface. After the washing operation with PBS (-), 2 mL of a 0.05% trypsin-0.05% EDTA solution (manufactured by Life Technologies Corp.) was added to the dish, the resulting mixture was heated in an incubator for 5 minutes, and the cells were peeled off from the dish. Using a phase-contrast microscope (device name: CKX41, manufactured by Olympus Corporation), it was verified that the cells had been peeled off. Then, 2 mL of NCS-containing DMEM was added to the dish to deactivate the trypsin. The cell suspension in the dish was transferred into one 15 ml centrifuge tube, and centrifuged (tradename: H-19FM, manufactured by KOKUSAN; at 200 ×g for 3 minutes). An aspirator was used to remove the supernatant. After the removal, 2 mL of NCS-containing DMEM was added to the centrifuge tube, and the resulting mixture was gently pipetted. The cells were dispersed to obtain a cell suspension. From the cell suspension, 100 µL thereof was taken out into an Eppendorf tube, and the sample was sucked into a cassette (tradename: Via1-Cassette, manufactured by ChemoMetec A/S). The number of cells was measured using a cell counter (tradename: NucleoCounter NC-3000, manufactured by ChemoMetec A/S), and the number of cells in the liquid was determined. Part of the cell suspension was transferred into a 15 ml centrifuge tube, and centrifuged (at 200 ×g for 3 minutes). A pipette was used to remove the supernatant. The first solution prepared in (1) was added to obtain a cell-containing first solution containing 2% Tetra-PEG-maleimidyl, and composed of a cell suspension having a cell concentration of 1 × 10⁷ cells/mL.

### (5) Production of Cell-containing Gel Particles

In a 35 mm dish, a polyester-made porous culture membrane having a diameter of 13 mm (tradename: ipCELLCULTURE Track Etched Membrane; the pore size, 0.45 µm; the pore density, 4E6 cm-2; the thickness, 12 µm; manufactured by it4ip S.A.) was placed, and 2 mL of the second solution was added to impregnate the membrane with the second solution. The cell-containing first solution prepared in (4) was fed into the liquid chamber of an inkjet head, from which droplets were dropped one by one onto the membrane. The cell-containing first solution was landed onto the second solution, and the two liquids reacted to produce cell-containing gel particles. After the gel was formed, the membrane was transferred to a 35 mm dish, into which NCS-containing DMEM was poured to make the gel particles float, and the gel particles were dispensed to a 96 well plate. The presence of gel particles in the wells was observed under a microscope. The plate was placed in a 37°C and 5% CO² environment in an incubator, and the cells were cultured for seven days.

### (6) Observation of Cell-containing Gel Particles

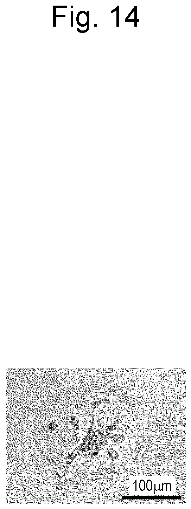

The cell-containing gel particles were observed under a phase-contrast microscope. As illustrated in FIG. 14, the results have verified that the 3T3 cells, which are adhesive cells, were successfully cultured in the gel. The state where the cells spread in the gel without directly adhering to the base plate was observed successfully.

### (Example 8) Production of Cell-containing Gel Particles Containing CHO Cell (Floating Cell)

### (1) Preparation of First Solution

This was performed in the same manner as in Example 7.

### (2) Preparation of Second Solution

This was performed in the same manner as in Example 7.

### (3) Culture of Cell

In an incubator, CHO (pMAM-luc) cells (JCRB Cell Bank, hereinafter referred to as "CHO") were cultured in a 100 mm dish for 72 hours, using Ham's F12 (tradename: F-12 Ham, manufactured by Sigma-Aldrich; hereinafter referred to as "Ham's F12") containing 10% fetal bovine serum (tradename: Fetal Bovine Serum, manufactured by GE Healthcare Inc.; hereinafter referred to as "FBS").

### (4) Production of Cell Suspension

To a dish, 5 mL of PBS (-) was added, and the PBS (-) was removed by suction using an aspirator to wash the surface. After the washing operation with PBS (-), 2 mL of a 0.05% trypsin-0.05% EDTA solution was added to the dish, the resulting mixture was heated in an incubator for 5 minutes, and the cells were peeled off from the dish. To the dish, 2 mL of FBS-containing Ham's F12 was added to deactivate the trypsin. The cell suspension in the dish was transferred into one 15 ml centrifuge tube, and centrifuged (at 200 ×g for 3 minutes). An aspirator was used to remove the supernatant. After the removal, 2 mL of FBS-containing Ham's F12 was added to the centrifuge tube. The resulting mixture was gently pipetted, and the cells were dispersed to obtain a cell suspension. From the cell suspension, 100 µL thereof was taken out into an Eppendorf tube, and the sample was sucked into a cassette. The number of cells was measured using a cell counter, and the number of cells in the liquid was determined. Part of the cell suspension was transferred into a 15 ml centrifuge tube, and centrifuged (at 200 ×g for 3 minutes). A pipette was used to remove the supernatant. The first solution prepared in (1) was added to obtain a cell-containing first solution containing 2% Tetra-PEG-maleimidyl, and composed of a cell suspension having a cell concentration of 1 × 10⁷ cells/mL.

### (5) Production of Cell-containing Gel Particles

This was performed in the same manner as in Example 7. The plate was placed in a 37°C and 5% CO₂ environment in an incubator, and the cells were cultured for five days.

### (6) Observation of Cell-containing Gel Particles

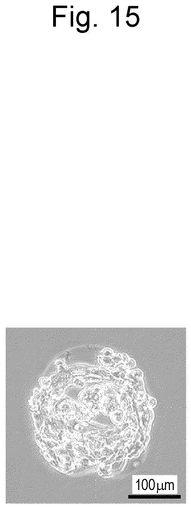

The cell-containing gel particles were observed under a phase-contrast microscope. As illustrated in FIG. 15, the results have verified that the CHO cells, which are floating cells, were successfully cultured in the gel.

### (Example 9) Evaluation of Cell Survival Rate

### (1) Staining of Cell

The cell-containing gel particles produced in Example 7 were used for evaluation. Propidium (manufactured by Sigma Aldrich; hereinafter referred to as "PI") and Hoechst33342 (H3570, manufactured by Thermo Fisher Scientific Inc.; hereinafter referred to as "Hoechst") were diluted with a culture medium so as to be at 0.5 ng/ml, and were incubated at 37°C. After the one-hour incubation, the cell-containing gel particles were observed under a fluorescence microscope (manufactured by Olympus Corporation) to obtain an image.

### (2) Calculation of Cell Survival Rate

On the basis of the image obtained in (1), the survival rate (%) was calculated in accordance with (the number of dead cells / the total number of cells) × 100, assuming that the number of cells stained with PI is the number of dead cells, and that the total number of cells stained with Hoechst is the total number of cells.

### (3) Criterion

The result that the cell survival rate was 75% or more on culture day 4 and culture day 7 of the cell-containing gel particles was rated A. The results are tabulated in Table 2. Table 2 verified that the cell survival rate was successfully maintained even though the cells were cultured in the gel particles for one week.

**[Table 2]**

| Culture Day | Day 4 | Day 7 |
|---|---|---|
| Rating | A | A |

### (Example 10) Production of Cell-containing Gel Particles Containing Neurocyte

### (1) Preparation of First Solution

This was performed in the same manner as in Example 7.

### (2) Preparation of Second Solution

This was performed in the same manner as in Example 7.

### (3) Production of Cell Suspension

Freeze-preserved glutamatergic neurocytes in a vial were thawed in a warm bath at 37°C, and then transferred to one 15 mL centrifuge tube, and 5 mL of DMEM was added to suspend the neurocytes. From the cell suspension, 100 µL thereof was taken out into an Eppendorf tube, and the sample was sucked into a cassette. The number of cells was measured using a cell counter, and the number of cells in the liquid was determined. The cell suspension in the 15 mL centrifuge tube was centrifuged (at 200 ×g for 3 minutes). An aspirator was used to remove the supernatant. The first solution and the second solution prepared in (1) and (2) were used to obtain a cell-containing first solution containing 1% Tetra-PEG-maleimidyl and 0.1% Thiol Gelatin, and composed of a cell suspension having a cell concentration of 1 × 10⁷ cells/mL.

### (4) Production of Cell-containing Gel Particles

This was performed in the same manner as in Example 7. The plate was placed in a 37°C and 5% CO₂ environment in an incubator, and the cells were cultured for seven days.

### (5) Immunofluorescent Staining of Cell-containing Gel Particles

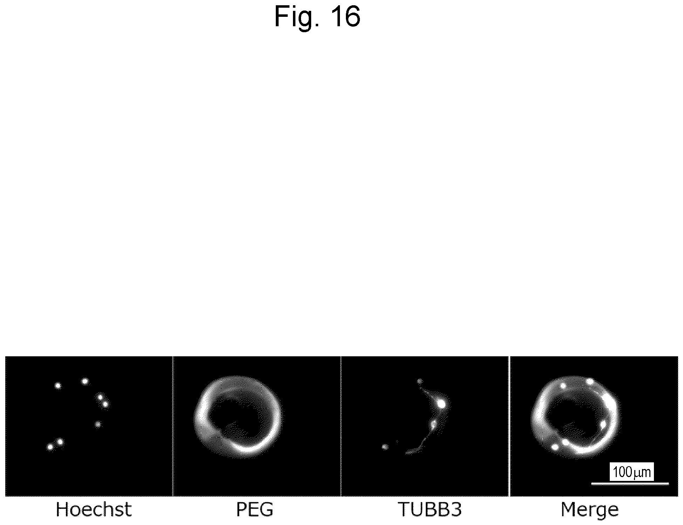

After the supernatant was removed, PBS (-) was added for washing (hereinafter referred to as "PBS washing"). After the supernatant was removed, 4% paraformaldehyde (tradename: 4% Paraformaldehyde Phosphate Buffer Solution, manufactured by Fujifilm Wako Pure Chemical Corporation; hereinafter referred to as "4% PFA") was added, and the resulting mixture was left to stand for 20 minutes to fix the cells. After the supernatant was removed, PBS washing was repeated three times. After the supernatant was removed, 0.1% Triton was added for 10-minute cell membrane permeation treatment. After the PBS washing was repeated three times, the cells were subjected to blocking with 1% BSA for one hour. A β3 tubulin antibody (tradename: β3-Tubulin (D71G9) XP Rabbit mAb #5568, manufactured by Cell Signaling Technology, Inc.; hereinafter referred to as "TUBB3") and a PEG antibody (tradename: Anti-Polyethylene glycol antibody [26A04] BSA and Azide free (ab94764), Abcam plc) were diluted with 1% BSA. After the blocking, the supernatant was removed, the primary antibody solution diluted was added, and the resulting mixture was incubated at 4°C overnight. The next day, PBS washing was repeated three times, the secondary antibody solution diluted with a 1% BSA solution was added, and the resulting mixture was incubated for one hour. The following operations were performed under a light shield of aluminum foil or the like. After the supernatant was removed, a Hoechst solution diluted with PBS was added, and the resulting mixture was subjected to nuclear stain for 5 minutes. PBS washing was repeated twice, and a mounting agent (tradename: Fluoromount, from DBS) was added dropwise. Observation under a fluorescence microscope (Carl Zeiss AG) verified that the cells in the gel particles on culture day 7 expressed TUBB3 which is a marker of a neurocyte (FIG. 16). In addition, the neurites were bent along the outline of the gel, thus allowing the observation of how the neurites remained in the gel.

### (Example 11) Size of Cell-containing Gel Particle

### (1) Preparation of First Solution

This was performed in the same manner as in Example 7.

### (2) Preparation of Second Solution

This was performed in the same manner as in Example 7.

### (3) Production of Cell Suspension

Freeze-preserved cardiomyocytes in a vial were thawed in a warm bath at 37°C, and then transferred to one 15 mL centrifuge tube, and 10 mL of DMEM was added to suspend the neurocytes. From the cell suspension, 100 µL thereof was taken out into an Eppendorf tube, and the sample was sucked into a cassette. The number of cells was measured using a cell counter, and the number of cells in the liquid was determined. Part of the cell suspension was dispensed from the 15 mL centrifuge tube, and centrifuged (at 200 ×g for 3 minutes). An aspirator was used to remove the supernatant. The first solution and the second solution prepared in (1) and (2) were used to obtain a cell-containing first solution containing 1% Tetra-PEG-maleimidyl and 0.1% Thiol Gelatin, and composed of a cell suspension having a cell concentration of 1 × 10⁷ cells/mL.

### (4) Production of Gel Particles

Gel particles having a diameter of 200 µm were produced in the same manner as in Example 7. To produce gel particles having a diameter of 2 mm, 1 µL of the second solution was dropped into a 35 mm dish, and into these droplets, 1 µL of the cell-containing first solution prepared in (3) was injected. After the gel was formed, DMEM was calmly added to a 35 mm dish. The dish was placed in a 37°C and 5% CO₂ environment in an incubator, and the cells were cultured for seven days.

### (5) Observation of Cell-containing Gel Particles

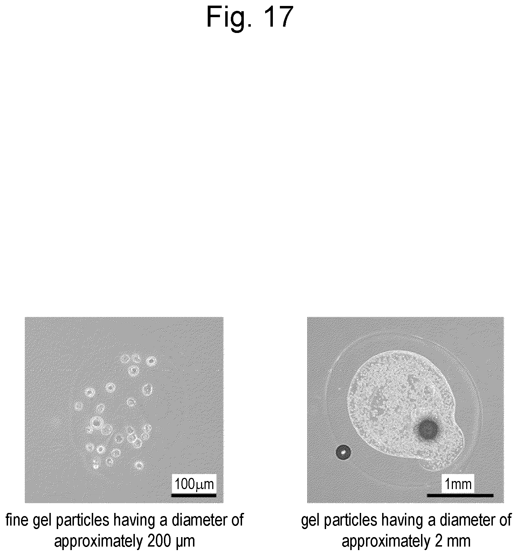

The cell-containing gel particles were observed under a phase-contrast microscope. The result has successfully verified, as illustrated in FIG. 17, that the cardiomyocytes were beating in the gel particles. In addition, it has successfully been verified that the individual cells were observed in the cell-containing gel particles having a diameter of 200 µm, but the individual cells were not observed in detail in the gel particles having a diameter of 2 mm.

### (Example 12) Cell-containing Gel Particle Containing One Cell

### (1) Preparation of First Solution

This was performed in the same manner as in Example 7.

### (2) Preparation of Second Solution

This was performed in the same manner as in Example 7.

### (3) Culture of Cell

This was performed in the same manner as in Example 7.

### (4) Production of Cell Suspension

In the same manner as in Example 7, a cell-containing first solution composed of a cell suspension having a cell concentration of 1 × 10⁶ cells/mL was obtained.

### (5) Production of Cell-containing Gel Particles

This was performed in the same manner as in Example 7.

### (6) Observation of Cell-containing Gel Particles

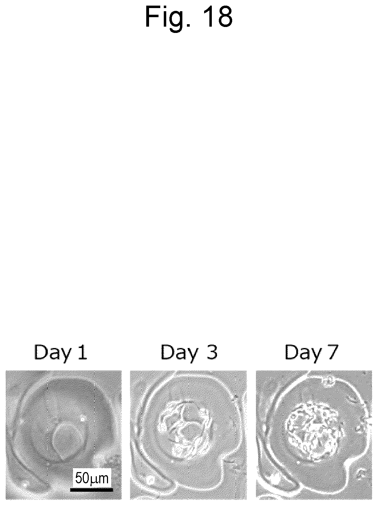

On culture days 1, 3, and 7, the cell-containing gel particles were observed under a phase-contrast microscope. Consequently, in the cell-containing gel particle containing one cell, cells proliferating were observed after 7 days, as illustrated in FIG. 18. This has successfully verified that even one cell can be cultured in the cell-containing gel particle.

### (Comparative Example 5) Cell-containing Gel Particles Containing Alginic Acid

The basic procedures were the same as in Example 7. However, the first solution and the second solution were produced using the following materials.

### (1) Preparation of First Solution

In 100 mL of ultrapure water, 0.584 g of calcium chloride (Model No.: 192-13925, manufactured by Wako Pure Chemical Industries, Ltd.) (hereinafter referred to as "CaCl₂") was dissolved to prepare a first solution composed of an aqueous solution of 100 mmol/L CaCl₂.

### (2) Preparation of Second Solution

In 2 mL of ultrapure water, 20 mg of sodium alginate (tradename: KIMICA ALGINSKAT-ONE, manufactured by Kimica Corporation) was dissolved to prepare an aqueous solution having a sodium alginate concentration of 1.0%.

### (3) Criterion for Particle Diameter

As the diameter of the cell-containing gel particle produced, 500 µm or less was rated A, and more than 500 µm was rated X. The results are tabulated in Table 3 below. As illustrated in Table 3, it has been verified that the 500 µm or less fine gel particles were successfully produced using sodium alginate.

### (4) Evaluation of Cell Survival Rate

This was performed in the same manner as in Example 9. As the cell survival rate on culture day 7, 75% or more was rated A, and less than 75% was rated X. The results are tabulated in Table 3 below. As illustrated in Table 3, it has been verified that it was not possible to maintain the cell survival rate in the fine alginic acid gel particles until one week later.

**[Table 3]**

| Alginic Acid | Particle Diameter | Cell Survival Rate |
|---|---|---|
| Rating | A | X |

### (Comparative Example 6) Cell-containing Gel Particles Containing Collagen

### (1) Preparation of Collagen Solution

A collagen solution was prepared using a collagen gel culture kit (Nitta Gelatin Inc.), and stored at 4°C.

### (2) Preparation of Cell Suspension

Using the collagen solution prepared in (1), a cell suspension having a cell concentration of 1 × 10⁶ cells/mL was prepared.

### (3) Production of Cell-containing Gel Particles

The cell suspension prepared in (2) was dropped 1 µL by 1 µL into a 35 mm dish, and allowed to gelate at 37°C for 10 minutes, and then, a culture medium was added. The gel particles were placed in a 37°C and 5% CO₂ environment in an incubator, and the cells were cultured for seven days.

### (4) Criterion for Particle Diameter

As the diameter of the cell-containing gel particle produced, 500 µm or less was rated A, and more than 500 µm was rated X. The results are tabulated in Table 4 below. As illustrated in Table 4, it has been verified that 500 µm or less fine gel particles were not successfully produced using collagen.

### (5) Evaluation of Cell Survival Rate

This was performed in the same manner as in Example 9. As the cell survival rate on culture day 7, 75% or more was rated A, and less than 75% was rated X. The results are tabulated in Table 4 below. As illustrated in Table 4, it has been verified that it was possible to maintain the cell survival rate in the collagen gel particles until one week later.

**[Table 4]**

| Collagen | Particle Diameter | Cell Survival Rate |
|---|---|---|
| Rating | X | A |

### (Comparative Example 7) Cell-containing Gel Particles Containing Gelatin (1) Preparation of Gelatin Solution

To 0.05 g of Thiol Gelatin, 1 ml of PBS (-) was added, and the gelatin was dissolved at 37°C overnight to prepare a 5% Thiol Gelatin solution.

### (2) Preparation of Cell Suspension

Using the gelatin solution prepared in (1), a cell suspension having a cell concentration of 1 × 10⁶ cells/mL was prepared.

### (3) Production of Cell-containing Gel Particles

The cell suspension prepared in (2) was dropped 1 µL by 1 µL into a 35 mm dish, and allowed to gelate at 4°C, and then, a culture medium was added. The gel particles were placed in a 37°C and 5% CO₂ environment in an incubator, and the cells were cultured for seven days.

### (4) Criterion for Particle Diameter

As the diameter of the cell-containing gel particle produced, 500 µm or less was rated A, and more than 500 µm was rated X. The results are tabulated in Table 5 below. As illustrated in Table 5, it has been verified that 500 µm or less fine gel particles were not successfully produced using gelatin.

### (5) Evaluation of Cell Survival Rate

The gel particles allowed to gelate at 4°C were cultured in an incubator at 37°C, with the result that the gel was melted, making it impossible to evaluate the survival rate of the cells cultured in the gel.

**[Table 5]**

| Gelatin | Particle Diameter | Cell Survival Rate |
|---|---|---|
| Rating | X | - |

### (Comparative Example 8) Cell-containing Gel Particles Containing PEG-SH

The basic procedures were the same as in Example 7. However, the second solution was produced using the following materials.

### (1) Preparation of Second Solution

Tetra-PEG-SH (tradename: SUNBRIGHT PTE-100SH, manufactured by Yuka Sangyo Co., Ltd.) was dissolved in PBS (-) to prepare a first solution containing 2% Tetra-PEG-SH.

### (2) Criterion for Particle Diameter

As the diameter of the cell-containing gel particle produced, 500 µm or less was rated A, and more than 500 µm was rated X. The results are tabulated in Table 6 below. As illustrated in Table 6, it has been verified that 500 µm or less fine gel particles were not successfully produced using collagen.

### (3) Evaluation of Cell Survival Rate

This was performed in the same manner as in Example 9. As the cell survival rate on culture day 7, 75% or more was rated A, and less than 75% was rated X. The results are tabulated in Table 6 below. As illustrated in Table 6, it has been verified that it was not possible to maintain the cell survival rate in the gel particles containing PEG-SH until one week later.

**[Table 6]**

| PEG-SH | Particle Diameter | Cell Survival Rate |
|---|---|---|
| Rating | A | X |

### (Example 13) Culture of Cell-containing Gel Particles Containing Floating Cells, under Adhesive Conditions

### (1) Preparation of First Solution

This was performed in the same manner as in Example 7.

### (2) Preparation of Second Solution

This was performed in the same manner as in Example 7.

### (3) Production of Cell Suspension

This was performed in the same manner as in Example 10 to obtain a cell-containing first solution containing 1% Tetra-PEG-maleimidyl and 0.1% Thiol Gelatin, and composed of a cell suspension containing an antibody-producing hybridoma (JCRB Cell Bank; hereinafter referred to as "HyB") at a cell concentration of 1 × 10⁷ cells/mL.

### (4) Production of Cell-containing Gel Particles

In a 35 mm dish, an 18 mm square cover glass (tradename: No.1 Thickness 0.13 to 0.17 mm, manufactured by Matsunami Glass Ind., Ltd.) was placed, and 1 mL of the second solution was added to coat the surface of the cover glass with the second solution. The cell-containing first solution prepared in (3) was fed into the liquid chamber of an ink jet head, from which droplets were dropped one by one onto the cover glass. The cell-containing first solution was landed onto the second solution, and the two liquids reacted to produce cell-containing gel particles. After the gel was formed, the cover glass was transferred to the 35 mm dish, and DMEM containing 10% FBS was calmly added. The dish was placed in a 37°C and 5% CO₂ environment in an incubator, and the cells were cultured for 7 days.

### (5) Observation of Cell-containing Gel Particles

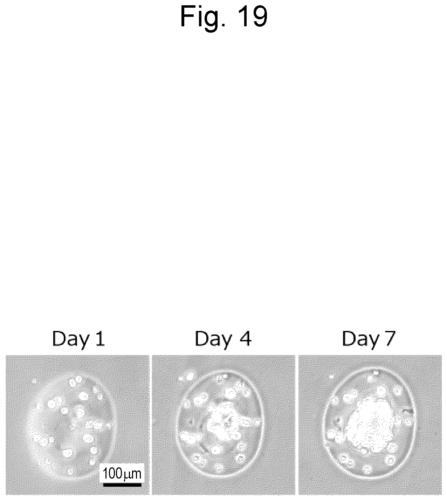

On culture days 1, 4, and 7, the cell-containing gel particles were observed under a phase-contrast microscope. As illustrated in FIG. 19, the result has successfully verified that culturing cells in the cell-containing gel particles adhering enabled the cells to be observed and tracked without being lost sight of.

### (6) Calculation of Cell Survival Rate

Staining with PI/Hoechst was performed in the same manner as in Example 9 to obtain an image of staining. The survival rate (%) was calculated in accordance with (the number of dead cells / the total number of cells) × 100, assuming that the number of cells stained with PI is the number of dead cells, and that the total number of cells stained with Hoechst is the total number of cells.

### (7) Criterion

The result that the cell survival rate was 75% or more on culture day 4 and culture day 7 of the cell-containing gel particles was rated A. The results are tabulated in Table 7. As illustrated in Table 7, it has been verified that, even when HyB, which was a floating cell, was cultured in the cell-containing gel particles allowed to adhere to a base plate, the cell survival rate was successfully maintained.

**[Table 7]**

| Culture Day | Day 4 | Day 7 |
|---|---|---|
| Rating | A | A |

### (Example 14) Measurement of Amount of IL-8 Secreted owing to LPS Stimulus

### (1) Preparation of First Solution

This was performed in the same manner as in Example 7.

### (2) Preparation of Second Solution

This was performed in the same manner as in Example 7.

### (3) Production of Cell Suspension

This was performed in the same manner as in Example 7 to obtain a cell-containing first solution containing 2% Tetra-PEG-maleimidyl and 0.1% Thiol Gelatin, and composed of a cell suspension containing HUVEC UmbilicalVein Endo Cells (Lonza Group AG; hereinafter referred to as "HUVEC") at a cell concentration of 1 × 10⁷ cells/mL.

### (4) Production of Cell-containing Gel Particles

The second solution was added to each well of a 96-well plate. The cell-containing first solution prepared in (3) was fed into the liquid chamber of an ink jet head, from which droplets were dropped nine by nine into the 96-well plate. The cell-containing first solution was landed onto the second solution, and the two liquids reacted to produce cell-containing gel particles. After the gel was formed, Endothelial Cell Growth Basal Medium-2 (Lonza Group AG: hereinafter referred to as EBM-2) was calmly added. The dish was placed in a 37°C and 5% CO₂ environment in an incubator, and the cells were cultured for two days.

### (5) LPS Treatment

The cell-containing gel particles were cultured for two days, and then lipopolysaccharide (hereinafter referred to as LPS) was added to the culture media so as to be at a concentration of 10 pg/mL, 100 pg/mL, and 10000 pg/mL. The particles were cultured for 24 hours. A well not treated with LPS was also produced as a control.

### (6) ELISA

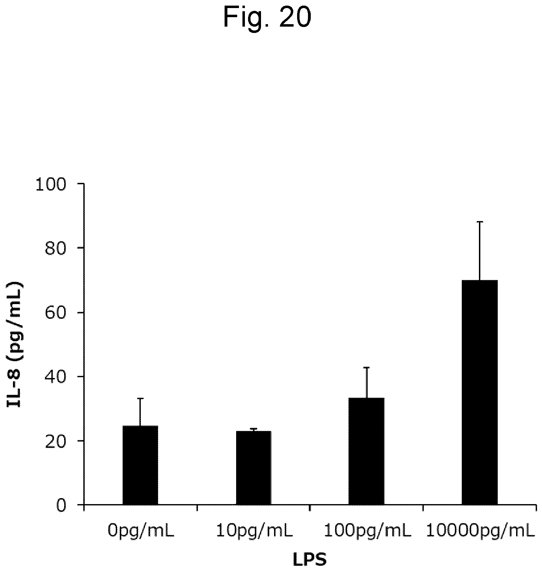

Using the culture supernatant of the cell-containing gel particles treated with LPS for 24 hours, the IL-8 was subjected to an ELISA. For the ELISA, an IL-8 Human Uncoated ELISA Kit (Invitrogen) was used, and the experiment was performed in accordance with the specified protocol. Using a plate reader, the absorbance at 450 nm was measured, and the IL-8 concentration of each sample was estimated from a standard, with the result that, as illustrated in FIG. 20, the amount of IL-8 in the supernatant was increased, depending on the LPS concentration.

### Description of the Reference Numeral

- 1: hydrogel
- 2: tetra-armed polymer
- 3: linear polymer
- 10: cell culture body
- 20: hydrogel
- 21: electrophilic functional group
- 30: cover glass
- 31: nucleophilic functional group
- 5: cell-containing gel particle
- 50: hydrogel
- 51: cell
- 60: first solution
- 61: second solution
- 62: cell-containing gel particle
- 63: culture container
- 64: culture medium
- 70: first solution
- 71: second solution
- 72: cell-containing gel particle
- 73: culture container
- 74: culture medium
- 8: cell-evaluating plate
- 80: well
- 81: cell-containing gel particle
- C: cell
All publications, patents, and patent applications cited herein are incorporated herein by reference in their entirety.

## Claims

1. A hydrogel comprising:
a multibranched polymer containing polyethylene glycol as a backbone, and containing one or more electrophilic functional groups or nucleophilic functional groups in at least one of a side chain and a terminal; and
a linear polymer containing two or more nucleophilic functional groups or electrophilic functional groups in at least one of a side chain and a terminal;
the multibranched polymer and the linear polymer being cross-linked with each other, wherein
the linear polymer is protein, peptide, or polysaccharide,
the electrophilic functional group(s) is/are one or more selected from the group consisting of maleimidyl, *N*-hydroxy-succinimidyl (NHS), sulfosuccinimidyl, phthalimidyl, imidazoyl, acryloyl, and nitrophenyl, and
the nucleophilic functional group(s) is/are one or more selected from the group consisting of thiol, amino, and -CO₂PhNO₂.

2. The hydrogel according to claim 1, wherein the linear polymer is gelatin.

3. The hydrogel according to claim 1 or 2, further comprising at least one cell.

4. The hydrogel according to claim 3, wherein the at least one cell comprises at least two or more species of cells.

5. The hydrogel according to claim 3, wherein the cell is a neurocyte.

6. An inkjet ink comprising:
a first solution comprising a multibranched polymer containing polyethylene glycol as a backbone, and containing one or more electrophilic functional groups or nucleophilic functional groups in at least one of a side chain and a terminal; and
a second solution including a linear polymer containing two or more nucleophilic functional groups or electrophilic functional groups in at least one of a side chain and a terminal, wherein
the linear polymer is protein, peptide, or polysaccharide,
the electrophilic functional group(s) is/are one or more selected from the group consisting of maleimidyl, *N-*hydroxy-succinimidyl (NHS), sulfosuccinimidyl, phthalimidyl, imidazoyl, acryloyl, and nitrophenyl, and
the nucleophilic functional group(s) is/are one or more selected from the group consisting of thiol, amino, and -CO₂PhNO₂.

7. The ink jet ink according to claim 6, wherein the linear polymer is gelatin.

8. The ink jet ink according to claim 6 or 7, wherein one or both of the first solution and the second solution comprises a cell.

9. The ink jet ink according to claim 8, wherein the cell is a neurocyte.

10. A method for producing a cell culture body, comprising:
retaining a first solution including a multibranched polymer containing polyethylene glycol as a backbone, and having one or more functional groups in at least one of a side chain and a terminal, any of the functional group(s) being one of a nucleophilic functional group and an electrophilic functional group; and
landing droplets of a second solution on the first solution to form one or more hydrogels, the droplets being discharged from a droplet-discharging device in such a manner that the second solution comes into contact with the first solution, the second solution including a linear polymer having one or more functional groups in at least one of a side chain and a terminal, any of the functional group(s) being the other one of the nucleophilic functional group and the electrophilic functional group, wherein
the linear polymer is protein, peptide, or polysaccharide,
the electrophilic functional group(s) is/are one or more selected from the group consisting of maleimidyl, *N-*hydroxy-succinimidyl (NHS), sulfosuccinimidyl, phthalimidyl, imidazoyl, acryloyl, and nitrophenyl,
the nucleophilic functional group(s) is/are one or more selected from the group consisting of thiol, amino, and -CO₂PhNO₂, and
at least one of the first solution and the second solution contains a cell.

11. The method for producing a cell culture body according to claim 10, wherein, when the first solution and the second solution are brought into contact with each other, at least one of the first solution and the second solution is discharged by an inkjet method.

12. The method for producing a cell culture body according to claim 11, wherein the first solution and the second solution are brought into contact with each other at a resolution of 500 µm or less.

13. A cell-containing gel particle comprising a hydrogel and a cell encapsulated in the hydrogel, the hydrogel comprising:
a multibranched polymer containing polyethylene glycol as a backbone, and containing one or more electrophilic functional groups or nucleophilic functional groups in at least one of a side chain and a terminal; and
a linear polymer containing two or more nucleophilic functional groups or electrophilic functional groups in at least one of a side chain and a terminal;
the multibranched polymer and the linear polymer being cross-linked with each other, wherein
the linear polymer is protein, peptide, or polysaccharide,
the electrophilic functional group(s) is/are one or more selected from the group consisting of maleimidyl, *N-*hydroxy-succinimidyl (NHS), sulfosuccinimidyl, phthalimidyl, imidazoyl, acryloyl, and nitrophenyl, and
the nucleophilic functional group(s) is/are one or more selected from the group consisting of thiol, amino, and -CO₂PhNO₂.

14. The cell-containing gel particle according to claim 13, wherein the linear polymer is gelatin.

15. The cell-containing gel particle according to claim 13 or 14, having a diameter of from 60 µm to 500 µm.

16. A method for producing a cell-containing gel particle, comprising mixing the following:
a first solution including a multibranched polymer containing polyethylene glycol as a backbone, and containing one or more electrophilic functional groups or nucleophilic functional groups in at least one of a side chain and a terminal; and
a second solution including a linear polymer containing two or more nucleophilic functional groups or electrophilic functional groups in at least one of a side chain and a terminal, wherein
the linear polymer is protein, peptide, or polysaccharide,
the electrophilic functional group(s) is/are one or more selected from the group consisting of maleimidyl, *N*-hydroxy-succinimidyl (NHS), sulfosuccinimidyl, phthalimidyl, imidazoyl, acryloyl, and nitrophenyl,
the nucleophilic functional group(s) is/are one or more selected from the group consisting of thiol, amino, and -CO₂PhNO₂,
one of the first solution and the second solution contains a cell, and
a droplet of one which is the first solution or the second solution, and contains the cell is mixed with the other one which is the first solution or the second solution, and does not contain the cell.

17. The method for producing a cell-containing gel particle according to claim 16, wherein the linear polymer is gelatin.

18. A method for culturing a cell, comprising: placing the cell-containing gel particle according to any one of claims 13 to 15 in a culture container; and culturing a cell in such a manner that the cell-containing gel particle is not in contact with the culture container.

19. A method for culturing a cell, comprising: allowing the cell-containing gel particle according to any one of claims 13 to 15 to adhere to a culture container; and culturing a cell in such a manner that the cell-containing gel particle is retained in the culture container.

20. A cell-evaluating plate comprising the cell-containing gel particle according to any one of claims 13 to 15.
